# FASCICULE DE BREVET EUROPEEN

(11) **EP 1 555 531 B1**
(45) Date de publication et mention de la délivrance du brevet: **07.03.2007**
(21) Numéro de dépôt: 05356011.6
(22) Date de dépôt: 14.01.2005
(51) Int. Cl.: G01N 33/68

(54) **Procédé d'évaluation de la dégradation du tissu cartilagineux d'un individu**
Verfahren zur Auswertung des Knorpelgewebeabbau
Method for evaluating cartilage tissue degradation

(30) Priorité: 14.01.2004 FR 0400292
(43) Date de publication de la demande: 20.07.2005
(73) Titulaire: Synarc, 69003 Lyon (FR)
(72) Inventeur: Garnero, Patrick, 69100 Villeurbanne (FR); Charni, Nadine, 69100 Villeurbanne (FR)
(74) Mandataire: Sarlin, Laure V.

(56) Documents cités:
- EP-A- 1 130 401
- WO-A-98/35235
- US-A- 6 132 976
- GARNERO PATRICK: "Osteoarthritis: biological markers for the future?" JOINT, BONE, SPINE : REVUE DU RHUMATISME. DEC 2002, vol. 69, no. 6, décembre 2002 (2002-12), pages 525-530, XP002298371 ISSN: 1297-319X

### SEQUENCE LISTING

<110> 11
   SYNARC
<120> Procédé d'évaluation de la dégradation du tissu cartilagineux d'un individu
<130> 1H715010-BEP-c1
<150> FR0400292
<151> 2004-01-14
<160> 12
<170> PatentIn version 3.1
<210> 1
   <211> 1418
   <212> PRT
   <213> Homo sapiens
<400> 1
   Met Ile Arg Leu Gly Ala Pro Gln Ser Leu Val Leu Leu Thr Leu Leu
   1               5                   10                  15
   Val Ala Ala Val Leu Arg Cys Gln Gly Gln Asp Val Arg Gln Pro Gly
               20                  25                  30
   Pro Lys Gly Gln Lys Gly Glu Pro Gly Asp Ile Lys Asp Ile Val Gly
           35                  40                  45
   Pro Lys Gly Pro Pro Gly Pro Gln Gly Pro Ala Gly Glu Gln Gly Pro
       50                  55                  60
   Arg Gly Asp Arg Gly Asp Lys Gly Glu Lys Gly Ala Pro Gly Pro Arg
   65 70 75 80
   Gly Arg Asp Gly Glu Pro Gly Thr Leu Gly Asn Pro Gly Pro Pro Gly
   85 90 95
   Pro Pro Gly Pro Pro Gly Pro Pro Gly Leu Gly Gly Asn Phe Ala Ala
   100 105 110
   Gln Met Ala Gly Gly Phe Asp Glu Lys Ala Gly Gly Ala Gln Leu Gly
   115 120 125
   Val Met Gln Gly Pro Met Gly Pro Met Gly Pro Arg Gly Pro Pro Gly
   130 135 140
   Pro Ala Gly Ala Pro Gly Pro Gln Gly Phe Gln Gly Asn Pro Gly Glu
   145 150 155 160
   Pro Gly Glu Pro Gly Val Ser Gly Pro Met Gly Pro Arg Gly Pro Pro
   165 170 175
   Gly Pro Pro Gly Lys Pro Gly Asp Asp Gly Glu Ala Gly Lys Pro Gly
   180 185 190
   Lys Ala Gly Glu Arg Gly Pro Pro Gly Pro Gln Gly Ala Arg Gly Phe
   195 200 205
   Pro Gly Thr Pro Gly Leu Pro Gly Val Lys Gly His Arg Gly Tyr Pro
   210 215 220
   Gly Leu Asp Gly Ala Lys Gly Glu Ala Gly Ala Pro Gly Val Lys Gly
   225 230 235 240
   Glu Ser Gly Ser Pro Gly Glu Asn Gly Ser Pro Gly Pro Met Gly Pro
   245 250 255
   Arg Gly Leu Pro Gly Glu Arg Gly Arg Thr Gly Pro Ala Gly Ala Ala
   260 265 270
   Gly Ala Arg Gly Asn Asp Gly Gln Pro Gly Pro Ala Gly Pro Pro Gly
   275 280 285
   Pro Val Gly Pro Ala Gly Gly Pro Gly Phe Pro Gly Ala Pro Gly Ala
   290 295 300
   Lys Gly Glu Ala Gly Pro Thr Gly Ala Arg Gly Pro Glu Gly Ala Gln
   305 310 315 320
   Gly Pro Arg Gly Glu Pro Gly Thr Pro Gly Ser Pro Gly Pro Ala Gly
   325 330 335
   Ala Ser Gly Asn Pro Gly Thr Asp Gly Ile Pro Gly Ala Lys Gly Ser
   340 345 350
   Ala Gly Ala Pro Gly Ile Ala Gly Ala Pro Gly Phe Pro Gly Pro Arg
   355 360 365
   Gly Pro Pro Asp Pro Gln Gly Ala Thr Gly Pro Leu Gly Pro Lys Gly
   370 375 380
   Gln Thr Gly Lys Pro Gly Ile Ala Gly Phe Lys Gly Glu Gln Gly Pro
   385 390 395 400
   Lys Gly Glu Pro Gly Pro Ala Gly Pro Gln Gly Ala Pro Gly Pro Ala
   405 410 415
   Gly Glu Glu Gly Lys Arg Gly Ala Arg Gly Glu Pro Gly Gly Val Gly
   420 425 430
   Pro Ile Gly Pro Pro Gly Glu Arg Gly Ala Pro Gly Asn Arg Gly Phe
   435 440 445
   Pro Gly Gln Asp Gly Leu Ala Gly Pro Lys Gly Ala Pro Gly Glu Arg
   450 455 460
   Gly Pro Ser Gly Leu Ala Gly Pro Lys Gly Ala Asn Gly Asp Pro Gly
   465 470 475 480
   Arg Pro Gly Glu Pro Gly Leu Pro Gly Ala Arg Gly Leu Thr Gly Arg
   485 490 495
   Pro Gly Asp Ala Gly Pro Gln Gly Lys Val Gly Pro Ser Gly Ala Pro
   500 505 510
   Gly Glu Asp Gly Arg Pro Gly Pro Pro Gly Pro Gln Gly Ala Arg Gly
   515 520 525
   Gln Pro Gly Val Met Gly Phe Pro Gly Pro Lys Gly Ala Asn Gly Glu
   530 535 540
   Pro Gly Lys Ala Gly Glu Lys Gly Leu Pro Gly Ala Pro Gly Leu Arg
   545 550 555 560
   Gly Leu Pro Gly Lys Asp Gly Glu Thr Gly Ala Glu Gly Pro Pro Gly
                   565                 570                 575
   Pro Ala Gly Pro Ala Gly Glu Arg Gly Glu Gln Gly Ala Pro Gly Pro
               580                 585                 590
   Ser Gly Phe Gln Gly Leu Pro Gly Pro Pro Gly Pro Pro Gly Glu Gly
           595                 600                 605
   Gly Lys Pro Gly Asp Gln Gly Val Pro Gly Glu Ala Gly Ala Pro Gly
       610                 615                 620
   Leu Val Gly Pro Arg Gly Glu Arg Gly Phe Pro Gly Glu Arg Gly Ser
   625                 630                 635                 640
   Pro Gly Ala Gln Gly Leu Gln Gly Pro Arg Gly Leu Pro Gly Thr Pro
                   645                 650                 655
   Gly Thr Asp Gly Pro Lys Gly Ala Ser Gly Pro Ala Gly Pro Pro Gly
               660                 665                 670
   Ala Gln Gly Pro Pro Gly Leu Gln Gly Met Pro Gly Glu Arg Gly Ala
           675                 680                 685
   Ala Gly Ile Ala Gly Pro Lys Gly Asp Arg Gly Asp Val Gly Glu Lys
       690                 695                 700
   Gly Pro Glu Gly Ala Pro Gly Lys Asp Gly Gly Arg Gly Leu Thr Gly
   705                 710                 715                 720
   Pro Ile Gly Pro Pro Gly Pro Ala Gly Ala Asn Gly Glu Lys Gly Glu
                   725                 730                 735
   Val Gly Pro Pro Gly Pro Ala Gly Ser Ala Gly Ala Arg Gly Ala Pro
               740                 745                 750
   Gly Glu Arg Gly Glu Thr Gly Pro Pro Gly Thr Ser Gly Ile Ala Gly
           755                 760                 765
   Pro Pro Gly Ala Asp Gly Gln Pro Gly Ala Lys Gly Glu Gln Gly Glu
       770                 775                 780
   Ala Gly Gln Lys Gly Asp Ala Gly Ala Pro Gly Pro Gln Gly Pro Ser
   785                 790                 795                 800
   Gly Ala Pro Gly Pro Gln Gly Pro Thr Gly Val Thr Gly Pro Lys Gly
                   805                 810                 815
   Ala Arg Gly Ala Gln Gly Pro Pro Gly Ala Thr Gly Phe Pro Gly Ala
               820                 825                 830
   Ala Gly Arg Val Gly Pro Pro Gly Ser Asn Gly Asn Pro Gly Pro Pro
           835                 840                 845
   Gly Pro Pro Gly Pro Ser Gly Lys Asp Gly Pro Lys Gly Ala Arg Gly
       850                 855                 860
   Asp Ser Gly Pro Pro Gly Arg Ala Gly Glu Pro Gly Leu Gln Gly Pro
   865                 870                 875                 880
   Ala Gly Pro Pro Gly Glu Lys Gly Glu Pro Gly Asp Asp Gly Pro Ser
                   885                 890                 895
   Gly Ala Glu Gly Pro Pro Gly Pro Gln Gly Leu Ala Gly Gln Arg Gly
               900                 905                 910
   Ile Val Gly Leu Pro Gly Gln Arg Gly Glu Arg Gly Phe Pro Gly Leu
           915                 920                 925
   Pro Gly Pro Ser Gly Glu Pro Gly Gln Gln Gly Ala Pro Gly Ala Ser
       930                 935                 940
   Gly Asp Arg Gly Pro Pro Gly Pro Val Gly Pro Pro Gly Leu Thr Gly
   945                 950                 955                 960
   Pro Ala Gly Glu Pro Gly Arg Glu Gly Ser Pro Gly Ala Asp Gly Pro
                   965                 970                 975
   Pro Gly Arg Asp Gly Ala Ala Gly Val Lys Gly Asp Arg Gly Glu Thr
               980                 985                 990
   Gly Ala Val Gly Ala Pro Gly Ala  Pro Gly Pro Pro Gly  Ser Pro Gly
           995                 1000                 1005
   Pro Ala  Gly Pro Thr Gly Lys  Gln Gly Asp Arg Gly  Glu Ala Gly
       1010                 1015                 1020
   Ala Gln  Gly Pro Met Gly Pro  Ser Gly Pro Ala Gly  Ala Arg Gly
       1025                 1030                 1035
   Ile Gln  Gly Pro Gln Gly Pro  Arg Gly Asp Lys Gly  Glu Ala Gly
       1040                 1045                 1050
   Glu Pro  Gly Glu Arg Gly Leu  Lys Gly His Arg Gly  Phe Thr Gly
       1055                 1060                 1065
   Leu Gln  Gly Leu Pro Gly Pro  Pro Gly Pro Ser Gly  Asp Gln Gly
       1070                 1075                 1080
   Ala Ser  Gly Pro Ala Gly Pro  Ser Gly Pro Arg Gly  Pro Pro Gly
       1085                 1090                 1095
   Pro Val  Gly Pro Ser Gly Lys  Asp Gly Ala Asn Gly  Ile Pro Gly
       1100                 1105                 1110
   Pro Ile  Gly Pro Pro Gly Pro  Arg Gly Arg Ser Gly  Glu Thr Gly
       1115                 1120                 1125
   Pro Ala  Gly Pro Pro Gly Asn  Pro Gly Pro Pro Gly  Pro Pro Gly
       1130                 1135                 1140
   Pro Pro  Gly Pro Gly Ile Asp  Met Ser Ala Phe Ala  Gly Leu Gly
       1145                 1150                 1155
   Pro Arg  Glu Lys Gly Pro Asp  Pro Leu Gln Tyr Met  Arg Ala Asp
       1160                 1165                 1170
   Gln Ala  Ala Gly Gly Leu Arg  Gln His Asp Ala Glu  Val Asp Ala
       1175                 1180                 1185
   Thr Leu  Lys Ser Leu Asn Asn  Gln Ile Glu Ser Ile  Arg Ser Pro
       1190                 1195                 1200
   Glu Gly  Ser Arg Lys Asn Pro  Ala Arg Thr Cys Arg  Asp Leu Lys
       1205                 1210                 1215
   Leu Cys  His Pro Glu Trp Lys  Ser Gly Asp Tyr Trp  Ile Asp Pro
       1220                 1225                 1230
   Asn Gln  Gly Cys Thr Leu Asp  Ala Met Lys Val Phe  Cys Asn Met
       1235                 1240                 1245
   Glu Thr  Gly Glu Thr Cys Val  Tyr Pro Asn Pro Ala  Asn Val Pro
       1250                 1255                 1260
   Lys Lys  Asn Trp Trp Ser Ser  Lys Ser Lys Glu Lys  Lys His Ile
   1265 1270 1275
   Trp Phe Gly Glu Thr Ile Asn Gly Gly Phe His Phe Ser Tyr Gly
   1280 1285 1290
   Asp Asp Asn Leu Ala Pro Asn Thr Ala Asn Val Gln Met Thr Phe
   1295 1300 1305
   Leu Arg Leu Leu Ser Thr Glu Gly Ser Gln Asn Ile Thr Tyr His
   1310 1315 1320
   Cys Lys Asn Ser Ile Ala Tyr Leu Asp Glu Ala Ala Gly Asn Leu
   1325 1330 1335
   Lys Lys Ala Leu Leu Ile Gln Gly Ser Asn Asp Val Glu Ile Arg
   1340 1345 1350
   Ala Glu Gly Asn Ser Arg Phe Thr Tyr Thr Ala Leu Lys Asp Gly
   1355 1360 1365
   Cys Thr Lys His Thr Gly Lys Trp Gly Lys Thr Val Ile Glu Tyr
   1370 1375 1380
   Arg Ser Gln Lys Thr Ser Arg Leu Pro Ile Ile Asp Ile Ala Pro
   1385 1390 1395
   Met Asp Ile Gly Gly Pro Glu Gln Glu Phe Gly Val Asp Ile Gly
   1400 1405 1410
   Pro Val Cys Phe Leu
   1415
<210> 2
   <211> 31
   <212> PRT
   <213> Artificial sequence
<220>
   <223> séquence issue de la partie 3/4 de la partie en triple hélice de la chaîne alpha-1 du collagène de type II
   <400> 2
   Gly Ser Ala Gly Ala Arg Gly Ala Pro Gly Glu Arg Gly Glu Thr Gly
   1 5 10 15
   Pro Pro Gly Thr Ser Gly Ile Ala Gly Pro Pro Gly Ala Asp Gly
   20 25 30
<210> 3
   <211> 11
   <212> PRT
   <213> Artificial Sequence
<220>
   <223> Helix-II
   <220>
   <221> MISC_FEATURE
   <222> (8)..(8)
   <223> Xaa is 4-hydroxyproline
<400> 3
   Glu Arg Gly Glu Thr Gly Pro Xaa Gly Thr Ser
   1 5 10
<210> 4
   <211> 6
   <212> PRT
   <213> Artificial sequence
<220>
   <223> CTX-II
   <400> 4
   Glu Lys Gly Pro Asp Pro
   1 5
<210> 5
   <211> 12
   <212> PRT
   <213> Artificial Sequence
<220>
   <223> Helix-II avec un acide aminé en plus en C-terminal
   <220>
   <221> MISC_FEATURE
   <222> (8)..(8)
   <223> Xaa is 4-hydroxyproline
<400> 5
   Glu Arg Gly Glu Thr Gly Pro Xaa Gly Thr Ser Gly
   1 5 10
<210> 6
   <211> 14
   <212> PRT
   <213> Artificial Sequence
<220>
   <223> Helix-II modifié en N-terminal
   <220>
   <221> MISC_FEATURE
   <222> (11)..(11)
   <223> Xaa is 4-hydroxyproline
<400> 6
   Ala Pro Gly Glu Arg Gly Glu Thr Gly Pro Xaa Gly Thr Ser
   1               5                   10
<210> 7
   <211> 15
   <212> PRT
   <213> Artificial Sequence
<220>
   <223> Helix-II modifié en C et N-terminal
   <220>
   <221> MISC_FEATURE
   <222> (11)..(11)
   <223> Xaa is 4-hydroxyproline
<400> 7
   Ala Pro Gly Glu Arg Gly Glu Thr Gly Pro Xaa Gly Thr Ser Gly
   1               5                   10                  15
<210> 8
   <211> 11
   <212> PRT
   <213> Artificial sequence
<220>
   <223> Analogue de Helix-II dans le collagène de type
   <220>
   <221> MISC_FEATURE
   <222> (8)..(8)
   <223> Xaa is 4-hydroxyproline
<400> 8
   Asp Arg Gly Glu Pro Gly Pro Xaa Gly Pro Ala
   1               5                   10
<210> 9
   <211> 11
   <212> PRT
   <213> Artificial Sequence
<220>
   <223> Helix-II avec une proline à la place d'une hydroxyproline
   <400> 9
   Glu Arg Gly Glu Thr Gly Pro Pro Gly Thr Ser
   1               5                   10
<210> 10
   <211> 10
   <212> PRT
   <213> Artificial sequence
<220>
   <223> Helix-II avec un acide aminé en moins du côté C-terminal
   <220>
   <221> MISC_FEATURE
   <222> (8)..(8)
   <223> Xaa is 4-hydroxyproline
<400> 10
   Glu Arg Gly Glu Thr Gly Pro Xaa Gly Thr
   1               5                   10
<210> 11
   <211> 9
   <212> PRT
   <213> Artificial sequence
<220>
   <223> Helix-II avec deux acides aminés en moins du côté C-terminal
   <220>
   <221> MISC_FEATURE
   <222> (8)..(8)
   <223> Xaa is 4-hydroxyproline
<400> 11
   Glu Arg Gly Glu Thr Gly Pro Xaa Gly
   1               5
<210> 12
   <211> 11
   <212> PRT
   <213> Artificial sequence
<220>
   <223> Analogue de Helix-II dans le collagène de type III
   <220>
   <221> MISC_FEATURE
   <222> (8)..(8)
   <223> Xaa is 4-hydroxypropline
<400> 12
   Glu Arg Gly Glu Thr Gly Pro Xaa Gly Pro Ala
   1               5                   10

## Description

Le domaine technique de l'invention est celui des marqueurs biologiques de la dégradation des tissus cartilagineux. La présente invention concerne plus particulièrement des procédés d'évaluation de la dégradation des tissus cartilagineux et leur mise en oeuvre, notamment, dans des méthodes de diagnostic, de suivi et de détermination d'un pronostic de pathologies des tissus cartilagineux.

Les pathologies des tissus cartilagineux se traduisent notamment par une dégradation des tissus cartilagineux. Le collagène de type II est le constituant protéique principal des tissus cartilagineux et est spécifique de ces tissus. Le collagène de type II est synthétisé par les chondrocytes sous forme de procollagène comprenant des propeptides N et C terminaux de part et d'autre du corps du collagène. Lors du clivage des propeptides qui se réalise naturellement in vivo lors de la maturation du collagène, le collagène mature sécrété par les chondrocytes a pour structure un domaine en triple hélice contenant trois chaînes α1 qui sont bordées du côté N et C terminal par trois chaînes telopeptidiques qui ne sont pas en triple hélice. Ces télopeptides ont pour fonction de pontage intramoléculaire entre les fibrilles de collagène.

La dégradation du collagène de type II est réalisée par des collagénases (MMP1, MMP8 et MMP13) (Billinghurst *et al*. Enhanced cleavage of type II collagen by collagenases in osteoarthritic articular cartilage. J. Clin. Invest. 1997, 99 : (1534-45). Un site caractéristique de coupure par les collagénases est situé dans le domaine en triple hélice du collagène de type II entre les résidus 775 et 776, qui génère 2 fragments de ¾ et ¼ de la longueur du collagène intact. Ces deux fragments ont un poids moléculaire de 75 et 25 kDa (pour une chaîne). Des anticorps reconnaissant la partie C-terminale du fragment Col2-3/4 et la partie N-terminale du fragment Col2-1/4 ont été développées (Hollander *et al*. Increased damage to type II collagen in osteoarthritic articular cartilage detected by a new immunoassay. J. Clin. Invest. 1994, 93: 1722-32). Il a été démontré que l'épitope du Col2-3/4 et non pas l'épitope du Col2-1/4 se retrouvait dans la circulation (Croucher and Hollander. Differential detection of type II collagen N-terminal and C-terminal denaturation epitopes in degrading cartilage. 1999. Mol. Pathol. 52, 323-31). Des immuno-dosages pour la détection de l'épitope du Col2-3/4 ont été mis au point (US patent 6132976). Des fragments plus petits du collagène de type II générés par des protéolyses successives peuvent être filtrés plus facilement dans les liquides biologiques, plus particulièrement lors de la filtration rénale.

L'un des objectifs de la présente invention est de fournir des procédés d'évaluation de la dégradation du collagène de type II utilisant de nouveaux marqueurs de sa dégradation et leur mise en oeuvre pour le diagnostic d'une pathologie des tissus cartilagineux, le suivi d'évolution d'une telle pathologie, le pronostic pré-symptomatique d'une telle pathologie, l'évaluation de l'efficacité d'un traitement thérapeutique administré pour lutter contre une pathologie des tissus cartilagineux, l'évaluation de la toxicité vis à vis des tissus cartilagineux d'un traitement thérapeutique et la prédétermination de l'évolution d'une pathologie des tissus cartilagineux chez un individu atteint d'une telle maladie.

Dans ce contexte, les inventeurs ont mis en évidence de nouveaux marqueurs biologiques spécifiques de la dégradation du collagène de type II. Ces marqueurs sont issus de la dégradation de la partie ¾ située dans la région en triple hélice de la chaîne α₁ du collagène de type II. Les inventeurs ont montré l'intérêt du dosage de tels marqueurs dans les liquides physiologiques, et en particulier dans les urines, pour le diagnostic et le suivi, notamment, de pathologies des tissus cartilagineux.

La présente invention a notamment pour objet un procédé d'évaluation de la dégradation des tissus cartilagineux d'un individu à partir de la détection *in vitro* dans un échantillon biologique provenant de l'individu, du niveau d'au moins un marqueur spécifique de la dégradation des tissus cartilagineux, le(s) marqueur(s) spécifique(s) détecté(s) étant un fragment de dégradation du collagène de type II, issus de la dégradation de la séquence en acides aminés **SEQ ID N°2** de la chaîne α₁ du collagène de type II située dans la région en triple hélice, ces dits fragments de dégradation comprenant au moins quatre acides aminés consécutifs présents dans le peptide Helix-II de séquence **SEQ ID N° 3,** éventuellement modifié post-traductionnellement et dans lequel la 4-hydroxyproline est éventuellement remplacée par une proline ou une 3-hydroxyproline.

Les différentes définitions données ci-après aident à comprendre la description de l'invention.

Par marqueur spécifique de la dégradation du collagène de type II, on entend un fragment protéique issu de la dégradation du collagène de type II et dont la détection permet d'être directement corrélée au niveau de dégradation du collagène de type II.

Par pathologie des tissus cartilagineux, on entend les désordres associés aux processus cataboliques des tissus cartilagineux, du cartilage de croissance et des disques intervertébraux. Les désordres associés au processus cataboliques des tissus cartilagineux sont par exemple des différents types d'arthrites comme la polyarthrite rhumatoïde, l'arthrose, l'arthrite psoriasique, la goutte, l'arthrite induite par *Yersinia*, l'arthrite pyrophosphate, l'arthrite septique ou les désordres liés aux disques intervertébraux comme les maladies dégénératives des disques ou les spondyarthropathies. Les désordres du cartilage de croissance incluent notamment la maladie de Kashin-Beck, l'acromégalie et le nanisme.

Par individu, on entend un animal ou, de préférence, un être humain, susceptible d'être atteint d'une pathologie des tissus cartilagineux, atteint d'une pathologie des tissus cartilagineux, qui est ou a été sous médication pour traiter une pathologie et notamment une pathologie des tissus cartilagineux.

Par échantillon biologique, on entend un liquide corporel ou fragment de tissu extrait d'un individu. Cet échantillon biologique peut être choisi parmi le sang, le sérum, le plasma, l'urine, la salive, la sueur, la lymphe, les larmes, le liquide synovial, les tissus, les cellules, les surnageants de cultures tissulaires ou cellulaires, et de préférence l'urine et le sérum.

Par stade destructeur d'une pathologie des tissus cartilagineux, on entend un stade de la pathologie dans lequel la dégradation des tissus cartilagineux peut être constatée par radiographie, résonance magnétique, résonance magnétique nucléaire, ultrasons, scanner, arthroscopie, biopsie tissulaire.

L'invention met en évidence que la détection dans un échantillon biologique d'un individu de certains fragments de dégradation du collagène de type II donne des informations spécifiques sur l'état de dégradation des tissus cartilagineux. Les inventeurs se sont particulièrement intéressés aux fragments de dégradation de la partie du collagène de type II de séquence **SEQ ID N°2.** Plus spécifiquement, les inventeurs se sont intéressés aux fragments de dégradation comprenant au moins quatre acides aminés consécutifs présents dans le peptide Helix-II de séquence **SEQ ID N° 3** et dans lequel la 4-hydroxyproline est éventuellement remplacée par une proline ou une 3-hydroxyproline. En particulier, l'invention utilise la détection des fragments de dégradation comprenant la séquence **SEQ ID N° 3** en totalité et encore plus particulièrement le fragment de dégradation de séquence **SEQ ID N°3,** nommé Helix-II. Les fragments de dégradation comprenant la séquence **SEQ ID N° 3** peuvent être définis par la formule générale suivante : (Xaa)m-ERGETGP-Hyp-GTS-(Xaa)n dans laquelle Hyp est la 4-hydroxyproline, m et n représentent indépendamment des entiers compris entre 1 et 10 et Xaa est un acide aminé ou dérivé d'acide aminé issu de la dégradation de la séquence **SEQ ID N°2**. Après traduction, le collagène de type II peut subir des modifications, lors de sa maturation. Par exemple, les acides aminés de la **SEQ ID N° 3** peuvent subir des modifications post-traductionnelles, telles que, entre autres, la citrullination (arginine devient citrulline), l'hydroxylation (proline devient l'hydroxyproline), la chloration (arginine ou lysine ou tyrosine deviennent chloro-argininine, chlorolysine et chlorotyrosine), la nitration (tyrosine devient la nitrotyrosine), la glycation (oxydation des lysines par des oses)...

Dans ce cas, l'invention visera à détecter les fragments de dégradation comprenant au moins quatre acides aminés consécutifs du peptide Helix-II mais dont un ou plusieurs acides aminés a été modifié post-traductionnellement.

De façon avantageuse, l'invention visera à détecter des fragments de dégradation de la séquence **SEQ ID N°2** du collagène de type II dont la partie C-terminale correspond aux 4 acides aminés C-terminaux du peptide Helix-II, à savoir Hyp-GTS, éventuellement modifiés post-traductionnellement, la sérine C terminale étant de préférence non modifiée post-traductionnellement. Selon un autre mode de réalisation particulier, l'invention visera à détecter des fragments de dégradation de la séquence **SEQ ID N°2** du collagène de type II, comprenant au moins 5 acides aminés consécutifs du peptide Helix-II de **SEQ ID N° 3**, dont éventuellement un ou plusieurs acides aminés a(ont) été modifié(s) post-traductionnellement et dans lequel la 4-hydroxyproline est éventuellement remplacée par une 3-hydroxyproline ou une proline.

Les méthodes de détection de la présence d'un marqueur utilisées selon l'invention peuvent être quantitatives ou qualitatives. Notamment, la détection peut être effectuée par une technique de chromatographie liquide à haute performance (HPLC), par fluorescence, par spectroscopie aux ultraviolets, par détection électrochimique ou par les techniques d'analyse protéomique ou « microarray ». De façon avantageuse, la détection consiste à mettre en contact l'échantillon biologique avec des anticorps, fragments d'anticorps ou analytes dirigés contre le marqueur à détecter, puis à détecter leur liaison avec le marqueur par une technique immunologique, de préférence choisie parmi la technique ELISA, les techniques immuno-enzymatiques, les techniques d'immunofluorescence, les techniques radio-immunologiques, les techniques chemo-immunologiques et les techniques d'analyse protéomique. Ces différentes techniques de détection sont bien connues de l'homme de l'art. Pour les techniques immunologiques, on pourra se référer à Diamandis et Cristopoulos, Immunoassay, Academic Press, San Diego 1996, notamment les pages 579 et suivantes. Pour les techniques d'analyse protéomique, on pourra notamment se référer à Urbanowska *et al*. Development of protein microarray technology to monitor biomarkers of rheumatoid arthritis disease. Cell Biol. Toxicol., 2003 19(3):189-202 et à Glokler and Angenendt. Protein and antibody microarray technology. Journal of chromatography B, 2003 797(1-2): 229-240.

Des anticorps monoclonaux, polyclonaux ou anticorps humanisés incluant les fragments Fc, Fab, les anticorps chimériques ou tout autre fragment d'anticorps antigène-spécifique pourront être utilisés. Selon un aspect de l'invention, on utilisera, pour la détection des fragments de dégradation, des anticorps se liant à un épitope dont au moins quatre, de préférence au moins cinq, acides aminés appartiennent au fragment de dégradation correspondant au peptide Helix-II de séquence **SEQ ID N°3,** ou au peptide Helix-II dont un ou plusieurs acides aminés a été modifié post-traductionnellement et préférentiellement à un épitope contenu dans la séquence **SEQ ID N° 3**, éventuellement modifiée post-traductionnellement. Par épitope, on entend le site situé sur la séquence peptidique avec lequel l'anticorps vient se lier. Un épitope est généralement constitué d'un enchaînement d'au moins deux acides aminés.

Dans ce contexte la présente invention a également pour objet les anticorps se liant à un épitope contenu dans les séquences **SEQ ID N° 3, SEQ ID N°5**, **SEQ ID N°6, SEQ ID N° 7** ou **SEQ ID N° 9,** notamment. Les anticorps se liant à un épitope contenu dans la séquence **SEQ ID N° 3** sont néanmoins préférés. En particulier, seront de préférence utilisés des anticorps se liant à un épitope dont la partie C-terminale est constituée par la séquence PGTS ou HypGTS, éventuellement modifiée post-traductionnellement, la sérine C terminale étant préférentiellement non modifiée. Selon un mode de réalisation particulier, les anticorps selon l'invention se lient à un épitope de séquence **SEQ ID N° 3**.

Les anticorps selon l'invention peuvent être monoclonaux ou, de préférence, polyclonaux. La production de ces anticorps se fait selon les méthodes standard, bien connues de l'homme du métier et décrites notamment par Harlow et Lane en 1988 et Köhler et Milstein en 1975 dans Continuous cultures of fused cells secreting antibody of predefined specificity. Nature. 1975, 256(5517):495-7. En particulier, on préparera par exemple selon les synthèses peptidiques classiques, les marqueurs à détecter que l'on couplera à une protéine dite « carrier » qui est préférentiellement la KLH (Keyhole limpet hemocyanine) ou l'albumine bovine ou humaine sérique, l'ovalbumine, la thyroglobuline ou toute autre protéine « carrier » comme l'edestine, la toxoïde tétanique ou cholérique, les polyaminoacides comme la poly-D-Lysine-D-acide glutamique. Ce couplage sera réalisé via une cystéine, une glutaraldéhyde, ou un carbodiimide par exemple. Cette séquence couplée permet alors l'immunisation de souris, rat, lapin, poulet, ou tout autre individu permettant la production d'anticorps.

Les anticorps au sens de l'invention seront qualifiés de spécifiques car ils reconnaissent les fragments de dégradation du collagène de type II contenant au moins 4 acides aminés consécutifs, en particulier au moins cinq, présents dans le peptide Helix-II de séquence **SEQ ID N° 3,** mais pas le collagène de type II intact.

La présente invention a également pour objet les trousses de dosage d'un fragment de dégradation du collagène de type II comportant un anticorps tel que défini ci-dessus. Ces trousses de dosage combinent des réactifs et instructions pour pouvoir mener le dosage du fragment de dégradation.

De plus, dans le cadre de l'invention, il a été montré l'intérêt de doser les marqueurs selon l'invention dans les urines pour le suivi, le diagnostic, le pronostic de pathologies des tissus cartilagineux et en particulier de l'arthrose, la polyarthrite rhumatoïde et la spondylarthrite. En effet, plus la pathologie est avancée, plus le niveau de marqueurs détectés est élevé, traduisant une augmentation de la dégradation du collagène de type II et donc des tissus cartilagineux. De plus, plus le risque de destruction du cartilage est élevé, plus le niveau de marqueurs détectés est élevé.

Plus précisément, la présente invention a également pour objet un procédé pour diagnostiquer une pathologie des tissus cartilagineux chez un individu mettant en oeuvre un procédé d'évaluation tel que défini ci-dessus. De façon avantageuse, le niveau de marqueurs spécifiques détectés est quantifié et comparé avec un niveau de référence caractéristique de l'absence de pathologie.

Selon un autre de ses aspects, la présente invention a pour objet un procédé pour suivre l'évolution d'une pathologie des tissus cartilagineux d'un individu atteint d'une pathologie des tissus cartilagineux mettant en oeuvre un procédé d'évaluation tel que défini précédemment. De façon avantageuse, le niveau de marqueurs spécifiques détectés est quantifié et comparé avec un niveau de référence, correspondant par exemple au niveau obtenu pour un stade prédéterminé de la pathologie.

Selon un autre de ses aspects, la présente invention a pour objet un procédé pour pronostiquer de façon pré-symptomatique une pathologie des tissus cartilagineux chez un individu mettant en oeuvre un procédé d'évaluation tel que défini précédemment. De façon avantageuse, le niveau de marqueurs spécifiques détectés est quantifié et comparé avec un niveau de référence établi chez des individus de même âge et de même sexe, sans pathologie ou à un stade prédéterminé de la pathologie.

Selon un autre de ses aspects, la présente invention a pour objet un procédé pour prédéterminer l'évolution d'une pathologie des tissus cartilagineux chez un individu atteint d'une telle maladie mettant en oeuvre un procédé d'évaluation tel que défini précédemment. De façon avantageuse, le niveau des marqueurs spécifiques détectés est quantifié et comparé avec un niveau de référence correspondant à un niveau seuil représentant l'absence de la pathologie ou représentant un stade prédéterminé de la pathologie.

Selon un autre de ses aspects, la présente invention a pour objet un procédé pour déterminer l'efficacité d'un médicament administré à un individu pour traiter une pathologie des tissus cartilagineux mettant en oeuvre un procédé d'évaluation tel que défini précédemment. De façon avantageuse, le niveau des marqueurs spécifiques détectés est quantifié et comparé avec un niveau de référence obtenu avant le début du traitement, ou avec un niveau obtenu chez des sujets sains.

Selon un autre de ses aspects, la présente invention a pour objet un procédé pour déterminer la toxicité vis à vis des tissus cartilagineux d'un traitement administré à un individu mettant en oeuvre un procédé tel que défini précédemment. De façon avantageuse, le niveau des marqueurs spécifiques détectés est quantifié et comparé avec un niveau de référence obtenu avant le début du traitement, ou avec un niveau obtenu chez des sujets sains.

Selon un autre de ses aspects, la présente invention a pour objet un procédé pour évaluer l'efficacité de traitements antiresorptifs, notamment biphosphonate, sur la dégradation du tissu cartilagineux chez un individu, mettant en oeuvre un procédé d'évaluation tel que défini précédemment.

Les résultats obtenus par les inventeurs ont également montré que les marqueurs selon l'invention étaient spécifiques de la dégradation du collagène de type II et qu'ils étaient en corrélation avec ceux obtenus avec un autre marqueur connu de séquence **SEQ ID N°4,** développé par Nordic Bioscience A/S, nommé CTX-II ou Cartilaps. De plus, la détection des marqueurs selon l'invention permet d'obtenir des informations complémentaires de celles obtenues avec d'autres marqueurs du cartilage, notamment le CTX-II. Par conséquent, au sens de l'invention, il est avantageux de détecter, dans l'échantillon biologique de l'individu, au moins deux marqueurs spécifiques de la dégradation du collagène de type II. En plus d'un marqueur selon l'invention, on détectera le marqueur de **SEQ ID N°4** ou tout autre marqueur de dégradation ou de la synthèse des tissus cartilagineux notamment ceux de la synthèse du collagène de type II c'est-à-dire des propeptides C-terminaux (appelé PIICP) ou N-terminaux (des formes IIA ou IIB du procollagène de type II appelé PIIANP et PIIBNP, Gamero *et al*. Uncoupling of type II collagen synthesis and degradation predicts progression of joint damage in patients with knee osteoarthritis. Arthritis Rheum., 2002 46(10) : 2613-24). En particulier, on détectera, dans l'échantillon biologique de l'individu, à la fois le niveau de Helix-II et le niveau de CTX-II de séquence **SEQ ID N°4**.

Les parties intitulées METHODES et RESULTATS donnés ci-après, en référence aux figures annexées, illustrent l'invention.
La **Fig. 1** est la courbe standard type de l'ELISA dosant le peptide Helix-II.
La **Fig. 2** illustre la spécificité des anticorps polyclonaux préparés vis-à-vis de l'Helix-II, par rapport à d'autres peptides synthétiques et vis-à-vis du collagène de type II humain intact et dénaturé à 80° C pendant 30 minutes.
La **Fig. 3** montre la corrélation entre 2 marqueurs de la dégradation des tissus cartilagineux chez 82 sujets contrôles et 89 patients atteints de polyarthrite rhumatoïde :
   - le marqueur de la chaîne α1 du collagène de type II: Helix-II
   - le marqueur du C- telopeptide du collagène de type II : CTX-II.
La **Fig. 4** montre la concentration en Helix-II (ng/mmol de créatinine urinaire) chez des adultes contrôles sains (n=85) chez des patients atteints d'arthroses du genou (n=90) et chez des patients atteints de polyarthrite rhumatoïde (n=89).
La **Fig. 5** montre le niveau de progression de la destruction des tissus cartilagineux en fonction de la combinaison de la mesure de l'excrétion urinaire de l'Helix-II et du CTX-II (n est le nombre de patients).
La **Fig. 6** montre la médiane des pourcentages de changement individuel du niveau urinaire d'Helix-II entre la visite de base et six mois après le début du traitement dans les deux groupes de femmes ménauposées, placebo et femmes traitées avec l'alendronate.
La **Fig. 7** montre la corrélation entre le logarithme népérien du niveau urinaire d'Helix-II (axe des x) et l'interligne articulaire de la hanche mesurée par radiographie (axe des y) chez des patients atteints d'arthrose de la hanche.
La **Fig. 8** montre un graphique en « boîte » représentant les taux urinaires d'Helix-II chez les patients atteints d'arthrose de la hanche (coxarthrose) avec une évolution lente ou rapide de leur maladie.
La **Fig. 9** montre une analyse immunohistochimique de sections de cartilage de patients atteints d'arthrose marquées avec l'antisérum Helix-II.

### A. METHODES

### 1. Préparation du peptide synthétique

La préparation des peptides synthétiques peut être réalisée selon les techniques largement décrites dans la littérature scientifique. Le peptide Helix-II de séquence **SEQ ID N°3,** dérivé de la séquence α-helix de la chaîne alpha I du collagène de type II (numéro d'accession dans GenBank : P02468) a été produit par la technique de synthèse peptidique sur phase solide en utilisant un blocage réversible de l'amine de l'acide aminé par le Fmoc (9-fluorénylméthyloxycarbonyl). La pureté du peptide a été vérifiée par HPLC et par spectrométrie de masse.

Le peptide servant d'immunogène, synthétisé avec une cystéine supplémentaire du côté *N*-terminal, a été couplé du côté *N*-terminal à une protéine porteuse qui est la KLH (keyhole limpet hemocyanine) ce qui va permettre une meilleure réponse immunologique vis-à-vis de ce peptide. Le peptide synthétique destiné à être adsorbé sur les microplaques a été couplé à la biotine (Harlow, E., Lane, D. Antibodies : a Laboratory Manual. Cold Spring Harbor, NY, Cold Spring Harbor Laboratory Press. 1988: 72-87).

### 2. Préparation des anticorps polyclonaux

Les lapins ont subi une injection par voie intra-péritonéale du peptide conjugué à la KLH émulsifié avec de l'adjuvant complet de Freud. Cette opération a été répétée 3 fois pendant trois mois en utilisant le même peptide avec de l'adjuvant incomplet de Freud. Après chaque injection, un échantillon de sang de lapin a été prélevé. On a laissé coaguler ce sang pendant 30 min et le tout a été centrifugé pendant 10 min à 2500 rpm pendant 10 min afin de récupérer le sérum. Dix jours après la dernière injection, les lapins ont été sacrifiés et tout le sang a été récupéré. Chaque prélèvement sérique a été titré par technique ELISA en présence du peptide de séquence **SEQ ID N°3** couplé à la biotine. L'antisérum avec le meilleur titre a été sélectionné pour la mise au point de l'ELISA de compétition (Harlow, E., Lane, D. Antibodies : a Laboratory Manual. Cold Spring Harbor, NY, Cold Spring Harbor Laboratory Press. 1988a: 92-120).

### 3. Titration des antisérums

Les titres des antisérums ont été réalisés en adsorbant le peptide conjugué à la biotine dilué avec du tampon de coating sur des plaques avidinées 2h à température ambiante. Après lavage, 100 µl d'antisérums dilué du 1/100 au 1/1000000 sont ajoutés à 100 µl de tampon de dilution dans les puits et sont mis à incuber pendant une nuit à 4°C. Après lavage, 100 µl de tampon d'anticorps secondaire couplé à la peroxydase et dirigé contre les immunoglobulines de lapins sont ajoutés pendant une heure à température ambiante. Les puits sont lavés et 100 µl de substrat (TMB) sont ajoutés. La réaction est stoppée avec 100 µl d'H₂SO₄.

### 4. ELISA de compétition pour le peptide α-helix de la chaîne alpha 1 du collagène de type II

Le peptide conjugué à la biotine dilué avec du tampon de coating est adsorbé sur des plaques avidinées 2h à température ambiante. Après lavage, 100 µl d'antisérums dilués au titre optimal sont ajoutés à 100 µl de standard (peptide de séquence **SEQ ID N°3** dilué dans du tampon) ou à 100 µl d'échantillons à doser et sont laissés à incuber pendant une nuit à 4°C. Après lavage, 100 µl de tampon d'anticorps secondaire couplé à la peroxydase et dirigé contre les immunoglobulines de lapins sont ajoutés pendant une heure à température ambiante. Les puits sont lavés et 100 µl de substrat (TMB) sont ajoutés. La réaction est stoppée avec 100 µl d'H₂SO₄.

### 5. Analyse immunohistochimique de sections de cartilage de genou arthrosique

Le cartilage a été obtenu d'un condyle fémoral d'un patient atteint d'arthrose du genou lors de la mise en place d'une prothèse. La technique utilisée est inspirée de l'article de Vankemmelbeke et al. (Vankemmelbeke M, Dekeyser PM, Hollander AP, Buttle DJ, Demeester J. Characterization of helical cleavages in type II collagen generated by matrixins. Biochem J. 1998 Mar 1;330 :633-40). Les coupes congelées de cartilage sont mises à incuber soit avec l'anticorps HELIX-II soit avec le sérum non-immun, dilué au 1/1000 durant 1 heure, puis sont rincées au PBS. Ensuite, les coupes sont mises à incuber durant 20 minutes avec l'anticorps biotinylé (« Dako LSAB ® 2 system » Dako, Carpinteria, CA), puis rincées au PBS. Une incubation de 20 minutes avec la streptavidine « Dako LSAB ® 2 system » (Dako, Carpinteria, CA) est réalisée, suivie d'un rinçage au PBS. Une incubation de 6 minutes avec le DAB (diaminobenzidine), suivie d'un rinçage à l'eau distillée sont alors réalisés. La coloration des coupes est réalisée par des bains successifs d'hématoxyline de Mayer (4 minutes) (Dako, Carpinteria, CA). Enfin, les lames sont immergées dans des bains successifs d'éthanol 70%, d'éthanol 95%, d'éthanol 100% et deux fois dans le toluène, en laissant 2 minutes par bain.

### 6. Patients atteints de polyarthrite rhumatoïde, d'arthrose, de la maladie de Paget et sujets contrôles

Nous avons sélectionné 89 patients qui présentaient les critères de polyarthrite rhumatoïde (PR) définis par l'American College of Rheumatology (Amett FC *et al*. The American Rheumatism Association 1987 revised criteria for classification of rheumatoid arthritis 1988; 31:315-324). Ces patients font partie d'une étude plus large multicentrique en double aveugle comparant l'efficacité de l'etanercept et du methotrexate chez 632 patients atteints de polyarthrite rhumatoïde débutante (Bathon JM *et al*. A comparison of etanercept and methotrexate in patients with early rheumatoid arthritis. N Engl J Med 2000 ; 343: 1586-1593 ; Gamero P *et al*. Association of Baseline Levels of Urinary Glucosyl-Galactosyl-Pyridinoline and Type II Collagen C-Telopeptide are associated with Progression of Joint Destruction in Patients with Early Rheumatoid Arthritis. Arthritis Rheum, 2002. 46(1):21-30). Les 89 patients avaient un âge supérieur à 18 ans, ne présentaient pas d'autres maladies, étaient atteints de PR depuis moins de trois ans et n'ont pas été traités avec le methotrexate. Afin de recruter les candidats à haut risque de progression, les patients devaient être en phase active de maladie c'est-à-dire lors de leur recrutement ces patients avaient plus de 12 articulations sensibles et plus de 10 articulations gonflées. Ces patients présentaient également un taux de facteur rhumatoïde positif et aussi 3 érosions osseuses visibles radiologiquement au niveau des mains, des poignets ou des pieds. Les traitements anti-rhumatismaux pris par les patients tels que l'hydroxychloroquinone et le sulfasalazine ont été interrompus 4 semaines avant le début de l'étude. Les traitements avec des doses fixes de corticostéroïdes et les anti-inflammatoires non stéroïdiens ont été permis. Les doses de corticosteroïdes ne pouvaient pas excéder 10 mg de prednisone par jour. Parmi les 89 patients de cette étude, 33 recevaient une dose de methotrexate qui s'échelonnait de 7,5 à 20 mg par semaine pendant les 8 premières semaines de l'étude, 28 recevaient 10 mg d'etanercept en sous-cutané 2 fois par semaine et 28 patients 25 mg, pendant 1 an. Les caractéristiques des patients atteints de PR au départ de l'étude sont présentées dans le **Tableau 1**. Pour tous les patients, la seconde miction urinaire a été collectée lors de la visite de base.

90 patients atteints d'arthrose selon les critères de l'ACR et appartenant à une étude randomisée ont été étudiés.
Les critères d'inclusion sont les suivants :
□ Hommes ou femmes adultes âgés de plus de 50 ans.
□ Confirmation clinique de l'arthrose du genou depuis au moins 3 mois avant l'étude et diagnostiqué selon les critères de l'American College of Rheumatology (ACR).

Les sujets sélectionnés avaient pour critères principaux d'inclusion :
- Douleur dans ou autour du genou la plupart du temps.
- raideur matinale > 30 minutes.
- des crépitations lors de mouvements actifs.
- Des ostéophytes du genou confirmés radiologiquement de grade 2 ou 3 (c'est-à-dire >1 et <4) selon l'échelle de Kellgren et de Lawrence
- Un score normalisé du score total du Western Ontario and MC Master

Universities (WOMAC) ≥50 mm sur une échelle analogue visuelle de 100 mm. En ce qui concerne les contrôles, les valeurs de référence des marqueurs biologiques ont été obtenues chez 82 sujets sains âgés de 26 à 90 ans (66% de femmes, moyenne d'âge 55 ± 15 ans). Tous ces individus étaient sains et n'étaient atteints d'aucune maladie et ne prenaient aucun traitement qui pouvait interférer le métabolisme de l'os ou de l'articulation incluant le traitement hormonal substitutif pour les femmes ménopausées.

**Tableau 1: Caractéristiques des patients avec polyarthrite rhumatoïde débutante**

| Femme (%) | 78 |
|---|---|
| Age (ans) | 49±12 |
| Durée de la maladie (mois) (médiane, 25; 75%) | 9 (3,3-19,8) |
| Facteur rhumatoïde (%) | 88 |
| Utilisateurs d'anti-inflammatoires non-stéroidiens (%) | 83 |
| Utilisateurs de corticoides (%) | 35 |
| Nombre d'articulations sensibles | 28±14 |
| Nombre d'articulations gonflées | 22±10 |
| Score d'évaluation du médecin | 5,8±1,9 |
| Score d'évaluation du patient | 6,4±1,8 |
| Index d'handicap ("Health Assessment Questionnaire") | 1,41±0,63 |
| Douleur (Echelle visuelle) | 5,8±2,2 |
| Protéine C-réactive (mg/dl) (médiane, 25; 75%) | 2,0 (05-4,8) |
| Score de Sharp * (médiane; 25; 75%) | |
| Total | 7,0 (2,0-15,0) |
| Erosion osseuse | 3,0 (0,0-7,8) |
| Pincement de l'interligne | 2,0 (0,0-7,8) |

| | |
|---|---|
| * Le score radiologique total de Sharp varie de 0 à 398; un score élevé représente une destruction des tissus cartilagineux plus importante. | |

### Patients atteints de la maladie osseuse de Paget

25 patients (5 femmes, 20 hommes ; moyenne de l'âge : 70± 7 ans) ont été sélectionnés. Le diagnostic de la maladie osseuse de Paget a été réalisé par rayon X et par scintigraphie osseuse et tous les patients ont dans le sérum une activité de la phosphatase alcaline deux fois supérieure à la limite supérieure de celle obtenue chez des adultes normaux. Aucun de ces patients n'a été traité avec la calcitonine durant les trois années précédent l'étude et avec les bisphosphonates durant les six mois précédent l'étude.

### 7. Evaluation de l'activité de la maladie lors de la visite de référence

L'évaluation du stade de la maladie chez les patients atteints de PR incluait un comptage complet des articulations sensibles et gonflées (71 articulations ont été vérifiées ; la hanche et les cervicales ont été vérifiées seulement pour leur sensibilité). Les articulations sont classées comme sensibles si une douleur apparaît lors de pression ou de mouvement de l'articulation. Les articulations sont classées comme gonflées si on remarque la présence de tissu mou enflé. Toutes les évaluations des articulations ont été réalisées par deux investigateurs indépendants et spécialement formés pour l'étude. Le questionnaire d'auto-évaluation clinique (Health Assessment Questionnaire, HAQ, Fries JF et al. The dimensions of health assessment questionnaire, disability and pain scores. J. Rheumatol 1982 ; 9 : 789-793 a été collecté pour tous les patients. Les autres indices de l'activité de la maladie incluaient l'évaluation par le patient et par le médecin (échelle allant de 0 pour le meilleur à 10 pour le pire), l'évaluation de la douleur par le patient (échelle allant de 0 pour le meilleur à 10 pour le pire) et le niveau sérique de la protéine C-réactive (CRP).

### 8. Mesure du niveau urinaire de CTXII

Le CTXII (décrit par Christgau *et al*. Bone 2001) urinaire a été mesuré par un ELISA de compétition (Cartilaps, Nordic Bioscience, Herlev, Danmark). Un anticorps monoclonal a été produit chez la souris et est dirigé contre la séquence EKGPDP du C-telopeptide du collagène de type II humain (Garnero *et al*., 2002 *supra* ; Garnero *et al*. Association of Baseline Levels of Markers of Bone and Cartilage Degradation are associated with Longterm Progression of Joint Damage in Patients with Early Rheumatoid Arthritis: the Cobra Study. Arthritis Rheum., 2002a 46(11):2847-56 ; Garnero *et al*. Uncoupling of type II collagen synthesis and degradation predicts progression of joint damage in patients with knee osteoarthritis. Arthritis Rheum. 2002b 46(10): 2613-24 ; Christgau *et al*. Collagen type II C-telopeptide fragments as an index of cartilage degradation. Bone 2001; 29:209-215). Cette séquence est spécifique du collagène II et n'est pas présente chez d'autres collagènes tels que le collagène de type I et chez des protéines structurales. Les coefficients de variation (CVs) *inter* et *intra* essai sont inférieurs à 8 et 10 % respectivement.

### 9. Evaluation radiologique des patients

Des radiographies antéro-postérieures des mains, poignets et pieds ont été faites lors de la visite de référence, à 6 mois et 12 mois chez tous les patients atteints de PR. Les radiographies de ces 89 patients ont été analysées par deux radiologues indépendants (Bathon *et al*., 2000 *supra*). Ce sont deux médecins spécialisés qui ont été formés à la méthode de comptage et procèdent au comptage en aveugle sans connaître le traitement pris par les patients ni la visite à laquelle a été prise la radiographie. La moyenne des comptages des deux radiologistes est utilisée pour estimer le pincement de l'interligne de l'articulation, l'érosion osseuse et le score total de Sharp qui est la somme du pincement de l'interligne et l'érosion osseuse. La moyenne du taux de progression par patient (unité de Sharp/an) est obtenue par régression linéaire des trois observations au même instant.

### 10. Analyses statistiques

Toutes les données sont exprimées en moyenne ± la déviation standard (DS). Du fait que les indices cliniques et radiologiques et les niveaux de marqueurs spécifiques de la dégradation du collagène de type II selon l'invention ou selon l'art antérieur ne sont pas normalement distribués, les corrélations ont été estimées par le coefficient de corrélation non paramétrique de rang de Spearman. La progression de la destruction de l'articulation a été définie par une augmentation du score radiographique de Sharp de 0,5 unité/an ou plus comme cela a été décrit précédemment (Gamero *et al*., 2002 *supra;* Gamero *et al*., 2002a *supra*). Les risques relatifs de la progression de la destruction de l'articulation en fonction des niveaux des marqueurs selon l'invention ou selon l'art antérieur ont été estimés par des analyses de régression logistique après ajustement de la prise du traitement.

Toutes les analyses statistiques ont été réalisées en utilisant le SAS (SAS institute Inc, Cary, NC) (SAS Institute Inc. SAS STAT Users's Guide, Version 6, 4^{th} Ed, Vol 1 and 2, Cary, NC SAS Institute, Inc).

### B. RESULTATS

### Exemple 1 : ELISA de compétition pour le peptide Helix-II de la chaîne alpha 1 du collagène de type II.

Les méthodes de production des peptides, des antisérums ainsi que la méthode ELISA de compétition sont décrites précédemment.

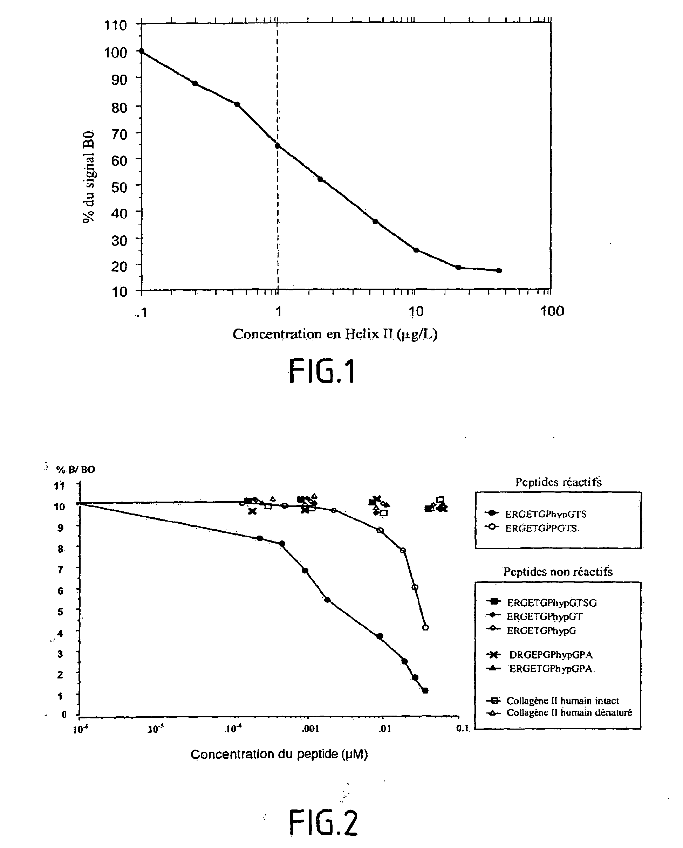

Une courbe standard ou de calibration a été obtenue sur un graphe semi log en dosant les 8 standards (de 0.25 à 40 µg/l) avec l'antisérum H12 sélectionné pour son titre (**Fig. 1**). Les valeurs sont exprimées en pourcentage de B0 qui est la valeur du standard 0 (sans peptide). La concentration en peptide Helix-II dans les échantillons est obtenue par extrapolation de la courbe de calibration.

La détection limite de cet essai a été déterminée par le calcul de la moyenne de 32 déterminations du standard 0 auquel on retranche trois fois la déviation standard. La détection limite est de 0,2 µg/l.

### Exemple 2 : Caractérisation de la spécificité de l'antisérum anti-Helix-II

L'antisérum sélectionné a été testé vis-à-vis de différents peptides selon la méthode ELISA décrite précédemment afin de vérifier sa spécificité. Les peptides testés sont : le peptide Helix-II de séquence **SEQ ID N°3** (le peptide immunogène), le peptide de séquence **SEQ ID N°9** (Helix-II où l'hydroxyproline a été remplacée par la proline), le peptide de séquence **SEQ ID N°5** correspondant au peptide immunogène avec un acide aminé supplémentaire du côté C-terminal, le peptide de séquence **SEQ ID N°10** correspondant au peptide immunogène avec un acide aminé en moins du côté C-terminal, le peptide de séquence **SEQ ID N°11** correspondant au peptide immunogène avec deux acide aminé en moins du côté C-terminal, les peptides de séquences **SEQ ID N°8** et **12** correspondant à la séquence homologue dans le collagène de type I et III humains respectivement. Comme le montre la **Figure 2,** seuls les peptides de séquences **SEQ ID N°3** et **SEQ ID N°9** sont reconnus par l'antisérum Helix-II mais avec moins d'affinité pour la séquence **SEQ ID N°9**. La spécificité de l'antiserum est donc en partie dépendante de la présence de l'hydroxyproline contenu dans la séquence **SEQ ID N°3**. Tous les autres peptides ainsi que le collagène de type II intact et dénaturé par la chaleur ne sont pas capables de rentrer en compétition avec le peptide Helix-II adsorbé sur la plaque. Donc l'antisérum H12 détecte un néoépitope provenant de la dégradation de la chaîne α1 du collagène de type II.

### Exemple 3 : caractéristiques analytiques de l'ELISA

Les performances analytiques de l'ELISA ont été vérifiées en effectuant :
- des tests de dilution : un échantillon urinaire est dosé pur et dilué (**Tableau** 2).
- des tests de recouvrement : on ajoute une quantité connue de peptide Helix-II à des échantillons urinaires (**Tableau 3**).
- des tests de précisions *intra* et *inter* essai : on mesure 32 fois le même échantillon (*intra*-essai) ou l'on mesure le même échantillon dans 10 manipulations différentes (*inter*-essai) afin de vérifier la variabilité des valeurs obtenues (**Tableau** 4).

**Tableau 2 : Tests de dilution de deux urines dans le dosage ELISA Helix-II**

| Echantillon | dilution | Helix-II (µg/L) | | Recouvrement (%) |
|---|---|---|---|---|
| | | Attendu | mesuré | |
| | pur | 2,784 | 2,784 | 100 |
| | 1/2 | 1,392 | 1,137 | 82 |
| I | 1/3 | 0,928 | 0,763 | 82 |
| | 1/4 | 0,696 | 0,592 | 85 |
| | 1/5 | 0,557 | 0,537 | 96 |
| | | | | |
| | pur | 4,126 | 4,126 | 100 |
| | 1/2 | 2,06 | 2,14 | 104 |
| II | 1/3 | 1,37 | 1,30 | 95 |
| | 1/4 | 1,03 | 1,065 | 103 |
| | 1/5 | 0,825 | 0,822 | 99,6 |

**Tableau 3 :Recouvrement de peptide standard dans le dosage ELISA du peptide Helix-II**

| échantillon | peptide hélicoïdal (µg/L) | | | Recouvrement(%) |
|---|---|---|---|---|
| | Endogène | Ajouté | Mesuré | |
| I | 1,337 | 1 | 1,069 | 91,52 |
| | 1,337 | 2 | 1,672 | 100,2 |
| | 1,337 | 5 | 4,015 | 126,7 |
| | 1,337 | 10 | 5,606 | 98,89 |
| II | 3,105 | 1 | 1,623 | 79,1 |
| | 3,105 | 2 | 2,854 | 112 |
| | 3,105 | 5 | 5,350 | 132,1 |
| | 3,105 | 10 | 10,06 | 153,6 |

**Tableau 4 : Précision de la mesure du peptide Helix-II intra et inter-essai dans trois échantillons urinaires.**

| *Intra*-essai | | | *Inter*-essai | |
|---|---|---|---|---|
| Echantillons | Concentration de Helix-II | CV, % | Concentration de Helix-II | CV, % |
| A | 0,570 ± 0,07 | 13,3 | 0,552 ± 0,08 | 14,9 |
| B | 1,350 ± 0,12 | 9,9 | 1,378 ± 0,152 | 11 |
| C | 1,949 ± 0,15 | 7,7 | 1,917 ± 0,14 | 7,6 |

Comme le montre le tableau 5, le niveau d'Helix-II reste stable après 24h d'incubation à température ambiante et à 4°C (bien qu'une urine sur les six testées montre une diminution de 19%). Similairement, quatre cycles répétés de congélation et décongélation n'affectent pas significativement le niveau d'Helix-II. Ces résultats indiquent une très bonne stabilité de la concentration d'Helix-II dans les urines.

**Tableau 5 : Stabilité du niveau d'Helix-II à température ambiante, à 4°C et après des cycles répétés de congélations-décongélations. Les données entre parenthèses montrent les niveaux d'Helix-II exprimés en pourcentage de changement par rapport aux niveaux initiaux.**

| Test de stabilité | échantillons | | HELIX-II urinaire (µg/L) | | | | | |
|---|---|---|---|---|---|---|---|---|
| | | | Temps d'incubations des urines (heures) | | | | | |
| | | | 0 | 2 | 4 | 6 | 24 | 48 |
| Température ambiante | I | | 3,60 | 3,66 | 3,64 | 3,23 | 3,56 (-1,1%) | ND |
| | II | | 1,67 | 1,95 | 1,62 | 1,76 | 1,71 (+2,4%) | ND |
| | III | | 2,46 | 2,35 | 2,20 | 2,10 | 2,00 (-19%) | ND |
| | IV | | 0,64 | ND | ND | ND | 0,69(+7,8%) | 0,73 |
| | V | | 2,62 | ND | ND | ND | 3,00 (+14,5%) | 3,00 |
| | VI | | 1,36 | ND | ND | ND | 1,60 (+17%) | 1,23 |
| | | | | | | | | |
| 4°C | I | | 3,60 | 4,30 | 3,28 | 3,42 | 3,56 (-1,1%) | ND |
| | II | | 1,67 | ND | 1,77 | 1,72 | 1,55 (-7,2%) | ND |
| | IIII | | 2,46 | 2,34 | 2,46 | 2,10 | 2,14 (-13%) | ND |
| | IV | | 0,64 | ND | ND | ND | 0,76 (+19%) | 0,76 |
| | V | | 2,62 | ND | ND | ND | 2,61 (-0,4%) | 3,06 |

| | | | Cycles congélation/décongélation | | | | | |
|---|---|---|---|---|---|---|---|---|
| | | | 0 | 1 | 2 | 3 | 4 | |
| | A | | 0,64 | 0,64 | 0,65 | 0,75 | 0,71 (+11%) | |
| congélation/ décongélation | B | | 1,36 | 1,36 | 1,41 | 1,24 | 1,24 (-8,8%) | |
| | C | | 4,68 | 5,01 | 4,91 | 4,10 | 5,35 (+14%) | |
| | D | | 1,81 | 1,99 | 2,08 | 2,07 | 2,02 (+11,6%) | |

| | | | | | | | | |
|---|---|---|---|---|---|---|---|---|
| ND: non disponible | | | | | | | | |

### Exemple 4: Corrélation linéaire entre deux marqueurs spécifiques de la dégradation des tissus cartilagineux : Helix-II et CTX-II

L'excrétion urinaire du marqueur Helix-II a été mesurée par ELISA (protocole précisé ci-dessus) et a été comparée avec l'excrétion urinaire de CTX-II mesurée également par ELISA (Cartilaps ELISA, Nordic Bioscience A/S). Chacun de ces marqueurs a été mesuré chez :
- 82 sujets contrôles
- 89 patients atteints de polyarthrite rhumatoïde.

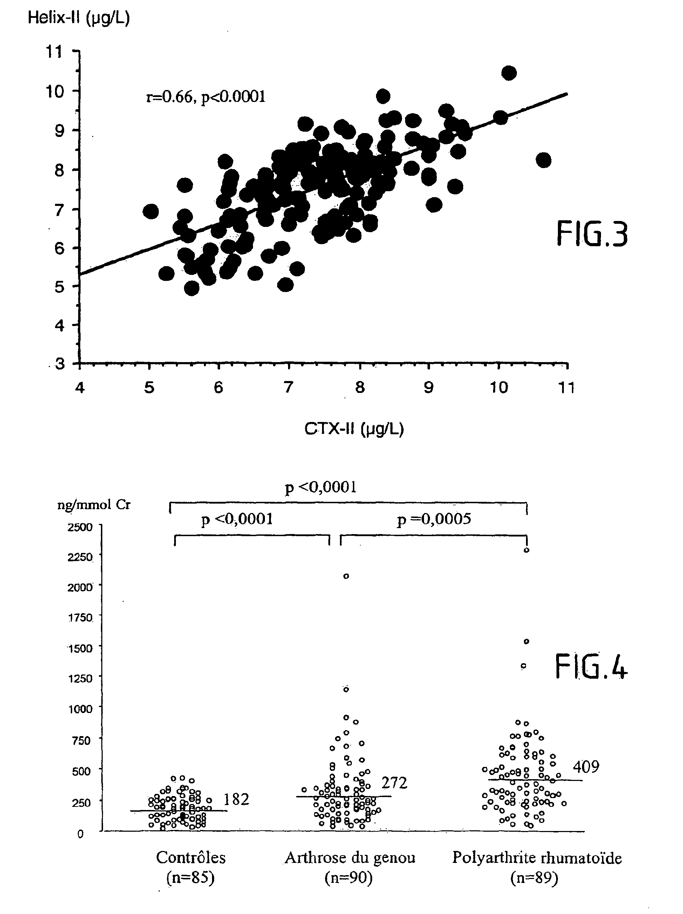

Les résultats (**Fig. 3**) montrent que le marqueur Helix-II corrèle significativement avec un marqueur connu spécifique de la pathologie ou de la dégradation des tissus cartilagineux le CTX-II ou Cartilaps (r=0,66, p<0,0001).

### Exemple 5 : Evaluation du niveau d'Helix-II chez des patients atteints d'arthrose du genou, de polyarthrite rhumatoïde et des contrôles.

Des urines de personnes contrôles ainsi que de patients atteints d'arthrose du genou et de polyarthrite rhumatoïde ont été collectées et dosées en Helix-II comme décrit dans le protocole (paragraphe A.4). La concentration en Helix-II (ng/mmol de créatinine urinaire) pour les différentes populations est présentée dans la **Fig. 4** : chez des adultes contrôles sains (n=85) chez des patients atteints d'arthroses du genou (n=90) et chez des patients atteints de polyarthrite rhumatoïde (n=89). Toutes les valeurs sont corrigées par la créatinine urinaire. Les lignes horizontales représentent la médiane de Helix-II/créatinine dans chaque groupe. Les valeurs de significativité de différence entre les groupes (p) sont représentées sur le graphe.

Sur la **Fig. 4**, la ligne horizontale pleine représente le niveau médian de chaque groupe. Lignes en pointillés représentent la limite supérieure des contrôles sains (95% percentile).

Les valeurs de p sont statistiquement significatives entre les groupes (tests de rang non paramétriques de Mann-Whitney rank). Le niveau d'Helix-II des patients atteints d'arthrose et de polyarthrite rhumatoïde est comparé à celui de sujets sains d'âge et de sexe similaire.

**Tableau 6 : Niveau urinaire d'Helix-II chez les patients atteints d'arthrose du genou, de polyarthrite rhumatoïde et de maladie de Paget de l'os et chez les contrôles sains.**

| Groupes | Niveau urinaire d'Helix-II (ng/mmol créatinine) | | p vs contrôles |
|---|---|---|---|
| | Médiane | 25 - 75 th percentile | |
| Contrôles (n=162) | 182 | 118-261 | |
| Arthrose du genou (n=90) | 272 | 169-378 | <0,0001 |
| Polyarthrite rhumatoïde (n=89) | 409* | 235-603 | <0,0001 |
| Maladie de Paget (n=25) | 218 | 158-302 | 0,87 |

| | | | |
|---|---|---|---|
| *p<0,0005 vs arthrose du genou | | | |

Le **Tableau 6** montre qu'il n'y a pas de différence significative entre le niveau d'Helix-II des patients atteints de maladie osseuse de Paget et les contrôles sains. Ces résultats indiquent qu'Helix-II dans les urines ne provient pas de la dégradation de l'os, car la maladie osseuse de Paget se caractérise par une dégradation très importante de l'os.

### Exemple 6 : Comparaison des deux marqueurs de la dégradation articulaire Helix-II et CTX-II

**Tableau 7 : Corrélation entre les niveaux de base de CTX-II et Helix-II et la progression de la destruction articulaire chez 89 patients avec polyarthrite récente**

| | Marqueur au temps de base | |
|---|---|---|
| Changement des scores radiographiques sur 1 an | Helix-II | CTX-II |
| | | |
| Erosion osseuse | 0,35* | 0,16 |
| | | |
| Score de Sharp total | 0,30** | 0,22*** |
| | | |

| | | |
|---|---|---|
| * p=0,002 , ** p=0,007, *** p=0,05 | | |

Lorsque les valeurs des marqueurs biologiques de la dégradation du collagène de type II (Helix-II et CTX-II (Cartilaps) urinaires) ainsi que la progression de la destruction articulaire évaluée par les changements des différents scores de Sharp sur un an, une corrélation (r de Spearman) significative a été observée entre des taux élevés de Helix-II et une progression plus rapide de l'érosion osseuse et de la destruction totale. Pour le CTX-II, l'association est plus faible et n'est observé que pour le score total de Sharp.

La destruction articulaire significative (> 0,5 unité/ an) des patients présentant des niveaux de marqueur dans le tertile supérieure par rapport aux autres patients est représenté dans le **Tableau 8**.

**Tableau 8 : Association entre les niveaux de base des marqueurs du cartilage (CTX-II et Helix-II) et le risque de progression de la destruction articulaire estimé par les changements radiologiques du score total de Sharp sur 1 an.**

| | Risque relatif de progression (intervalle de confiance à 95%) | |
|---|---|---|
| | Helix-II | CTX-II |
| | | |
| Non ajusté | 6,9 (2,5- 19,6) | 3,1 (1,2-7,8) |
| | | |
| -Ajusté par le score de Sharp | 6,6 (2,3-18,8) | 3,1 (1,2-7,9) |
| | | |
| -Ajusté par la CRP | 6,1 (2,1-17,8) | 2,2 (0,8-6,0) |
| | | |
| -Ajusté par le score Sharp et CRP | 5,9 (2,0- 17,2) | 2,2 (0,8-86,3) |

Tous les risques relatifs ont été ajustés pour chaque groupe de traitement.

L'intérêt clinique des marqueurs biologiques comme facteurs prédictifs de la progression, est combiné avec l'identification de valeur seuil permettant au clinicien d'identifier les patients à risque important de progression. Dans l'étude ci-dessus, il a été montré que les patients qui, au temps de base, avaient un taux de Helix-II dans le tertile supérieur de la population (33% de la population avec les niveaux les plus élevés) avaient en moyenne un risque relatif de progression de destruction articulaire 6,9 fois plus élevé que les patients qui avaient des taux dans les deux tertiles inférieurs. De façon importante, ce risque augmenté de progression pour des taux élevés d'Helix-II est en partie indépendant du niveau d'autres facteurs de risque et notamment le niveau de destruction articulaire initial (évalué par le score de Sharp) et le niveau d'inflammation systémique évalué par la concentration sérique de la protéine C-réactive (CRP). Pour le CTX-II, l'association est aussi significative, avec toutefois un risque relatif plus faible et non-significatif après ajustement avec la CRP. Tous les risques relatifs ont été calculés par régression logistique

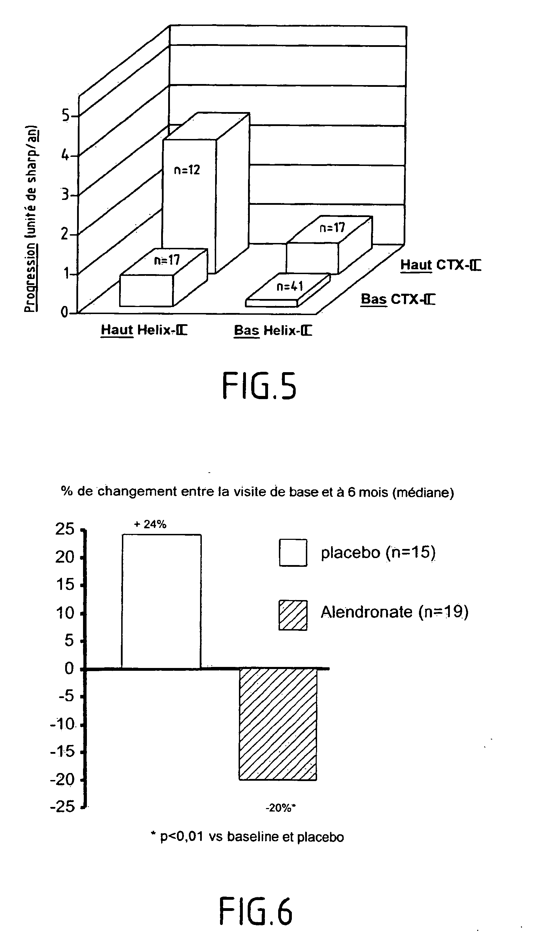

Comme le montre la **Fig.5**, la combinaison de la mesure de l'excrétion urinaire de l'Helix-II et du CTX-II, permet une meilleure identification des patients avec polyarthrites récente à progression rapide. Dans cette étude, les 87 patients PR ont été séparés en quatre groupe sur la base de leur taux initiaux d'Helix-II et de CTX-II en utilisant comme valeur seuil pour chaque marqueur le tertile supérieur («haut Helix-II et CTX-II). Les patients qui ont des taux d'Helix-II et de CTX-II dans le tertile supérieur, ont une progression de la destruction articulaire évaluée par le changement du score total de Sharp beaucoup plus importante que les patients présentant soit des taux élevés d'Helix-II soit des taux élevés de CTX-II. Ces résultats confirment donc que le dosage urinaire de Helix-II reflète des mécanismes de destruction articulaire indépendants du CTX-II dans la PR. La combinaison de ces deux marqueurs améliore la prédiction de la destruction articulaire.

### Exemple 7 : Effet du traitement par l'Alendronate (10 mg/jour) sur l'excrétion urinaire d'Helix-II chez des femmes ménopausées avec de l'ostéoporose.

**Tableau 9 : Valeurs médianes d'Helix-II urinaire obtenues à la visite de base et six mois après le début du traitement dans le groupe placebo et le groupe traité à l'alendronate**

| Mediane (25; 75 %) | | Placebo (n=15) | | | Alendronate (n=19) | |
|---|---|---|---|---|---|---|
| | | temps 0 | 6 mois | | Temps 0 | 6 mois |
| HELIX-II (ng/mmol Cr) | | 180 (85-203) | 214 (159-272) | | 258 (122-328) | 130 (112-193)* |

| | | | | | | |
|---|---|---|---|---|---|---|
| * p< 0,01 par rapport au temps 0 et aux taux dans le groupe placebo. | | | | | | |

34 femmes ménopausées ont été sélectionnées au hasard à partir d'une plus grande étude clinique incluant un total de 500 femmes elles-mêmes randomisées. Pour être randomisées dans cette étude, les femmes doivent être âgées de 60 à 90 ans et leur densité minérale osseuse de la colonne vertébrale ou de la hanche doit être inférieure d'au moins de 2,5 fois la déviation standard de la moyenne obtenue chez des jeunes femmes saines (c'est-à-dire avec une ostéoporose selon les critères de l'OMS). Ces patientes ostéoporotiques sont randomisées en deux groupes celui du placebo et celui de l'alendronate (10 mg/J) et sont traitées pendant 6 mois.

La seconde miction urinaire du matin a été prélevée à jeun durant la visite de base et six mois après le début du traitement afin de mesurer le niveau urinaire d'HELIX-II. Le **Tableau 9** montre les valeurs médianes d'Helix-II urinaire obtenues à la visite de base et six mois après le début du traitement dans les deux groupes. La **figure 6** montre la médiane du pourcentage de changement individuel entre la visite de base et six mois après le début du traitement dans les deux groupes, placebo et femmes traitées avec l'alendronate. L'alendronate à une dose de 10mg/jour produit une diminution significative du niveau d'HELIX-II à 6 mois en comparaison avec la visite de base et avec la diminution observée chez les femmes recevant un placebo.

Ces données sont en accord avec les résultats obtenus dans le modèle animal de l'arthrose montrant un effet "chondroprotecteur" de l'alendronate (Hayami T, Pickarski M, Wesolowski GA, McLane J, Bone A, Destefano J, Rodan GA, Duong le T. The role of subchondral bone remodeling in osteoarthritis: reduction of cartilage degeneration and prevention of osteophyte formation by alendronate in the rat anterior cruciate ligament transection model. Arthritis Rheum. 2004 Apr;50(4):1193-206.

### Exemple 8 : Le niveau d'Helix-II urinaire est augmenté chez des patients étant atteints d'une arthrose de la hanche avec destruction rapide.

L'étude cas contrôle regroupe 40 patients (23 femmes, 17 hommes, âge moyen : 64 ans (± 12 ans)) qui présentent les critères de l'American College of Rheumatology de l'arthrose primitive de la hanche. Ces patients sont venus consulter pour une douleur de la hanche entre 1996 et 1999. Deux radiographies de la hanche ont été réalisées à un an d'intervalle chez ces patients et les échantillons d'urine ont été collectés lors de la deuxième radiographie afin de mesurer le niveau d'Helix II. Douze de ces patients présentaient les critères d'une rapide progression de l'arthrose de la hanche et 28 ont été sélectionnés à cause de leur lente progression du pincement de l'interligne de l'articulation de la hanche.

La progression rapide de l'arthrose de la hanche a été définie par les 5 critères suivants :
- une douleur importante de la hanche
- des symptômes depuis au moins deux ans
- une progression du pincement de l'interligne de l'articulation de la hanche supérieur à 1 mm par an
- un taux de sédimentation des érythrocytes <20 mm/1^{ère} heure
- et une absence d'inflammation ou de cristaux induisant l'affection de l'articulation selon les critères proposés par Lequesne et Ray (Lequesne M, Ray G. La coxopathie destructice rapide idiopathique. Rev Rhum 1989 ; 56 :115-119).

Les 28 autres patients ont une progression plus lente de l'arthrose de la hanche défini par une perte de l'espace interarticulaire <0,20 mm durant une année.

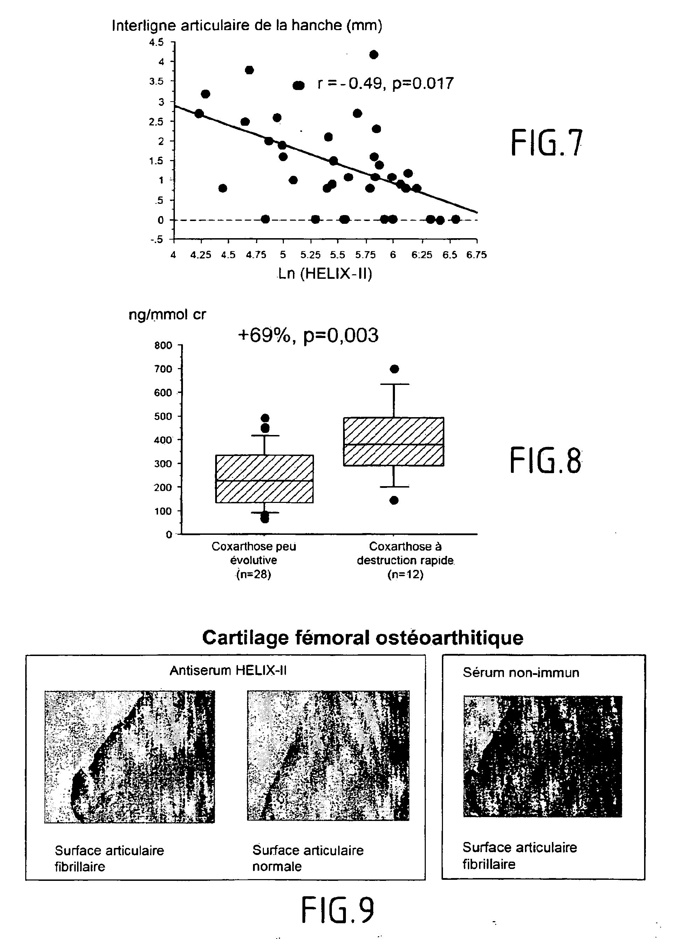

**La Fig. 7** est une corrélation entre le logarithme népérien du niveau urinaire d'Helix-II (axe des x) et l'interligne articulaire de la hanche mesurée par radiographie (axe des y) chez des patients atteints d'arthrose de la hanche.

**La Fig. 8** est un graphique en « boîte » montrant les taux urinaires d'Helix-II chez les patients atteints d'arthrose de la hanche (coxarthrose) avec une évolution lente ou rapide de leur maladie. De bas en haut, la "boîte" montre le 25^{ème}, le 50^{ème} (médiane) et le 75^{ème} percentile des valeurs d'Helix-II, alors que les limites des lignes de part et d'autre de la "boite" indiquent le 10^{ème} et 90^{ème} percentile, respectivement.

Les patients ayant une coxarthrose à destruction rapide ont un niveau médian d'Helix-II supérieur de 69% par rapport aux patients à coxarthrose peu évolutive (p=0,003, par un test non apparié et non paramétrique de Mann-Witney). Lorsque tous le patients atteints de coxarthrose sont regroupés, une augmentation du niveau d'Helix-II est associée à une diminution de l'interligne articulaire de la hanche (r = - 0,49, p = 0,017) mesurée par radiographie

### Exemple 9

Quand les sections de cartilage du patient arthrosique sont mises à incuber en présence de l'antisérum Helix-II (**Fig. 9**), qui montre une analyse immunohistochimique de sections de cartilage de patients atteints d'arthrose, une coloration apparaît sur la surface fibrillaire du cartilage alors que sur le même cartilage une section d'une zone non affectée ne présente pas de coloration. Les sections de cartilage contrôles mis à incuber avec le sérum non-immun montrent un très léger bruit du fond. Ces expérimentations suggèrent que le marqueur Helix-II est spécifique de la dégradation du cartilage osteoarthritique.

## Revendications

1. - Procédé d'évaluation de la dégradation des tissus cartilagineux d'un individu à partir de la détection *in vitro* dans un échantillon biologique provenant de l'individu, du niveau d'au moins un marqueur spécifique de la dégradation des tissus cartilagineux **caractérisé en ce que** les marqueurs spécifiques détectés sont des fragments de dégradation du collagène de type II, issus de la dégradation de la séquence en acides aminés **SEQ ID N°2** de la chaîne α₁ du collagène de type II située dans la région en triple hélice, ces dits fragments de dégradation comprenant au moins quatre acides aminés consécutifs présents dans le peptide Helix-II de séquence **SEQ ID N° 3,** éventuellement modifié post-traductionnellement, et dans lequel la 4-hydroxyproline est éventuellement remplacée par une proline ou une 3-hydroxyproline.

2. - Procédé d'évaluation selon la revendication 1 **caractérisé en ce que** les marqueurs spécifiques détectés sont des fragments de dégradation du collagène de type II, issus de la dégradation de la séquence en acides aminés **SEQ ID N°2** de la chaîne α₁ du collagène de type II située dans la région en triple hélice, ces dits fragments de dégradation comprenant au moins quatre acides aminés consécutifs présents dans le peptide Helix-II de séquence **SEQ ID N° 3,** éventuellement modifié post-traductionnellement.

3. - Procédé d'évaluation selon la revendication 1 ou 2, **caractérisé en ce que** l'on détecte le niveau de fragments de dégradation de la **SEQ ID N°2** du collagène de type II dont la partie C-terminale est constituée des 4 acides aminés C-terminaux du peptide Helix-II, à savoir Hyp-GTS, éventuellement modifiés post-traductionnellement, la sérine C terminale étant de préférence non modifiée post-traductionnellement.

4. - Procédé d'évaluation selon l'une des revendications 1 à 3, **caractérisé en ce que** l'on détecte le niveau de fragments de dégradation de la **SEQ ID N°2** du collagène de type II, comprenant au moins 5 acides aminés consécutifs du peptide Helix-II de **SEQ IN N° 3,** dont éventuellement un ou plusieurs acides aminés a(ont) été modifié(s) post-traductionnellement et dans lequel la 4-hydroxyproline est éventuellement remplacée par une 3-hydroxyproline ou une proline.

5. - Procédé d'évaluation selon l'une des revendications 1 à 4, **caractérisé en ce que** l'on détecte le niveau d'au moins un fragment de dégradation du collagène de type II, dont la séquence comprend la séquence **SEQ ID N°3**.

6. - Procédé d'évaluation selon l'une des revendications 1 à 5, **caractérisé en ce que** l'on détecte le niveau de Helix-II de séquence **SEQ ID N°3.**

7. - Procédé d'évaluation selon l'une des revendications 1 à 6, **caractérisé en ce que** la détection est effectuée par une technique de chromatographie liquide à haute performance (HPLC), par fluorescence, par spectroscopie aux ultraviolets, par détection électrochimique ou par des techniques d'analyse protéomique.

8. - Procédé d'évaluation selon l'une des revendications 1 à 6, **caractérisé en ce que** la détection consiste à mettre en contact l'échantillon biologique avec des anticorps, fragments d'anticorps ou analytes dirigés contre le marqueur à détecter, puis à détecter leur liaison avec le marqueur par une technique immunologique, de préférence choisie parmi la technique ELISA, les techniques immuno-enzymatiques, les techniques d'immunofluorescence, les techniques radio-immunologiques, les techniques chemo-immunologiques ou les techniques d'analyse protéomique.

9. - Procédé d'évaluation selon la revendication 8, **caractérisé en ce que** l'échantillon biologique est mis en contact avec des anticorps polyclonaux.

10. - Procédé d'évaluation selon la revendication 8, **caractérisé en ce que** l'échantillon biologique est mis en contact avec des anticorps monoclonaux.

11. **-** Procédé d'évaluation selon l'une des revendications 1 à 10 **caractérisé en ce que** l'échantillon biologique est choisi parmi le sang, le sérum, le plasma, l'urine, la salive, la sueur, la lymphe, les larmes, le liquide synovial, les tissus, les cellules, ou les surnageants de cultures tissulaires ou cellulaires de l'individu.

12. - Procédé d'évaluation de la dégradation du tissu cartilagineux d'un individu à partir de la détection, *in vitro,* dans un échantillon biologique de l'individu, de la présence de fragments de dégradation du collagène de type II, **caractérisé en ce que** l'on met en contact l'échantillon biologique avec des anticorps se liant à un épitope contenu dans la séquence **SEQ ID N° 3.**

13. **-** Procédé d'évaluation selon la revendication 12, **caractérisé en ce que** les anticorps mis en oeuvre sont polyclonaux.

14. - Procédé d'évaluation selon la revendication 12, **caractérisé en ce que** les anticorps mis en oeuvre sont monoclonaux.

15. - Procédé d'évaluation selon l'une des revendication 12 à 14, **caractérisé en ce que** la liaison anticorps/épitope est détectée par une technique immunologique choisie parmi la technique ELISA, les techniques immuno-enzymatiques, les techniques d'immunofluorescence, les techniques radio-immunologiques, les techniques chemo-immunologiques et les techniques d'analyse protéomique.

16. - Procédé d'évaluation selon l'une des revendications 12 à 14, **caractérisé en ce que** l'échantillon biologique est choisi parmi le sang, le sérum, le plasma, l'urine, la salive, la sueur, la lymphe, les larmes, le liquide synovial, les tissus, les cellules, ou les surnageants de cultures tissulaires ou cellulaires de l'individu.

17. - Procédé pour diagnostiquer une pathologie des tissus cartilagineux chez un individu mettant en oeuvre un procédé d'évaluation selon l'une des revendications 1 à 16.

18. - Procédé selon la revendication 17, **caractérisé en ce que** le niveau de marqueurs spécifiques détectés est quantifié et comparé avec un niveau de référence caractéristique de l'absence de pathologie.

19. - Procédé pour suivre l'évolution d'une pathologie des tissus cartilagineux d'un individu atteint d'une pathologie des tissus cartilagineux mettant en oeuvre un procédé d'évaluation selon l'une des revendications 1 à 16.

20. - Procédé selon la revendication 19, **caractérisé en ce que** le niveau de marqueurs spécifiques détectés est quantifié et comparé avec un niveau de référence, correspondant par exemple au niveau obtenu pour un stade prédéterminé de la pathologie.

21. **-** Procédé pour pronostiquer de façon pré-symptomatique une pathologie des tissus cartilagineux chez un individu mettant en oeuvre un procédé d'évaluation selon l'une des revendications 1 à 16.

22. - Procédé selon la revendication 21, **caractérisé en ce que** le niveau de marqueurs spécifiques détectés est quantifié et comparé avec un niveau de référence établi chez des individus de même âge et de même sexe, sans pathologie ou à un stade prédéterminé de la pathologie.

23. **-** Procédé pour prédéterminer l'évolution d'une pathologie des tissus cartilagineux chez un individu atteint d'une telle maladie mettant en oeuvre un procédé d'évaluation selon l'une des revendications 1 à 16.

24. - Procédé selon la revendication 23, **caractérisé en ce que** le niveau des marqueurs spécifiques détectés est quantifié et comparé avec un niveau de référence correspondant à un niveau représentant l'absence de la pathologie ou représentant un stade prédéterminé de la pathologie.

25. **-** Procédé selon l'une des revendications 17 à 24, **caractérisé en ce que** l'on détecte, dans l'échantillon biologique de l'individu, au moins deux marqueurs spécifiques de la dégradation du collagène de type II.

26. - Procédé selon la revendication 23 **caractérisé en ce que** l'on détecte, dans l'échantillon biologique de l'individu, à la fois le niveau de Helix-II et le niveau de CTX-II de séquence **SEQ ID N°4.**

27. - Procédé selon l'une des revendications 17 à 26, **caractérisé en ce que** la pathologie des tissus cartilagineux est un rhumatisme inflammatoire, une arthropathie métabolique, un rhumatisme dégénératif, la polyarthrite rhumatoïde, une spondyarthropathie ou l'arthrose.

28. - Procédé selon la revendication 27 **caractérisé en ce que** la pathologie des tissus cartilagineux est la polyarthrite rhumatoïde.

29. **-** Procédé selon la revendication 27 **caractérisé en ce que** la pathologie des tissus cartilagineux est l'arthrose.

30. - Procédé selon la revendication 27 **caractérisé en ce que** la pathologie des tissus cartilagineux est la spondylarthrite.

31. **-** Anticorps se liant à un épitope dont au moins quatre, de préférence au moins cinq, acides aminés appartiennent au fragment de dégradation correspondant au peptide Helix-II de séquence **SEQ ID N°3**, ou au peptide Helix-II dont un ou plusieurs acides aminés a été modifié post-traductionnellement et préférentiellement à un épitope contenu dans la séquence **SEQ ID N° 3**.

32. - Anticorps selon la revendication 31 se liant à un épitope dont la partie C-terminale est constituée par PGTS ou HypGTS, éventuellement modifiés post-traductionnellement, la sérine C terminale étant préférentiellement non modifiée.

33. - Anticorps se liant à un épitope contenu dans les séquences **SEQ ID N° 3, SEQ ID N°5, SEQ ID N°6, SEQ ID N°7,** ou **SEQ ID N°9.**

34. - Anticorps selon l'une des revendications 31 à 33 se liant à un épitope dont la séquence correspond à **SEQ ID N° 3.**

35. - Anticorps selon l'une des revendications 31 à 34, **caractérisé en ce qu'**il est polyclonal.

36. **-** Anticorps selon l'une des revendications 31 à 34, **caractérisé en ce qu'**il est monoclonal.

37. - Trousse de dosage d'un fragment de dégradation du collagène de type II comportant un anticorps selon l'une des revendications 31 à 36.

## Claims

1. A method for assessing the degradation of cartilaginous tissues of an individual from *in vitro* detection in a biological sample from the individual, of the level of at least one specific label of the degradation of cartilaginous tissues, **characterized in that** the detected specific labels are degradation fragments of collagen of type II, originating from the degradation of the amino acid sequence SEQ ID No.2 of the α₁ chain of collagen of type II located in the triple helix region, said degradation fragments comprising at least four consecutive amino acids present in the Helix-II peptide of sequence SEQ ID No.3, possibly modified post-translationally, and in which a 4-hydroxyproline is possibly replaced with a proline or 3-hydroxyproline.

2. The assessment method according to claim 1, **characterized in that** the detected specific labels are degradation fragments of collagen of type II, originating from the degradation of the amino acid sequence SEQ ID No.2 of the α₁ chain of collagen of type II, located in the triple helix region, said degradation fragments comprising at least 4 consecutive amino acids present in the Helix-II peptide of sequence SEQ ID No.3, possibly modified post-translationally.

3. The assessment method according to claim 1 or 2, **characterized in that** the level of degradation fragments of SEQ ID No.2 of collagen of type II is detected, for which the C-terminal portion consists of 4 C-terminal amino acids of the Helix-II peptide, i.e., Hyp-GTS, possibly modified post-translationally, the C-terminal serine being preferably not modified post-translationally.

4. The assessment method according to any of claims 1 to 3, **characterized in that** the level of degradation fragments of SEQ ID No.2 of collagen of type II comprising at least 5 consecutive amino acids of the Helix-II peptide of SEQ ID No.3 is detected, for which one or more amino acids have possibly been modified post-translationally and in which hydroxyproline is possibly replaced by a 3-hydroxyproline or proline.

5. The assessment method according to any of claims 1 to 4, **characterized in that** the level of at least one degradation fragment of collagen of type II is detected, the sequence of which comprises sequence SEQ ID No.3.

6. The assessment method according to any of claims 1 to 5, **characterized in that** the level of Helix-II of sequence SEQ ID No.3 is detected.

7. The assessment method according to any of claims 1 to 6, **characterized in that** detection is carried out by a high performance liquid chromatography (HPLC), fluorescence, ultraviolet spectroscopy technique, by electro-chemical detection or by proteomic analysis techniques.

8. The assessment method according to any of claims 1 to 6, **characterized in that** the detection consists of putting the biological sample into contact with antibodies, antibody fragments or analytes directed against the label to be detected, and then of detecting their binding with the label by an immunological technique, preferably selected from the ELISA technique, immuno-enzymatic techniques, immuno-fluorescence techniques, radio-immunological techniques, chemo-immunological techniques or proteomic analysis techniques.

9. The assessment method according to claim 8, **characterized in that** the biological sample is put into contact with polyclonal antibodies.

10. The assessment method according to claim 8, **characterized in that** the biological sample is put into contact with monoclonal antibodies.

11. The assessment method according to any of claims 1 to 10, **characterized in that** the biological sample is selected from blood, serum, plasma, urine, saliva, sweat, lymph, tears, synovial liquid, tissues, cells, or supernatants of tissue or cell cultures from the individual.

12. A method for assessing the degradation of cartilaginous tissue of an individual from *in vitro* detection in a biological sample from the individual, of the presence of degradation fragments of collagen of type II, **characterized in that** the biological sample is put into contact with antibodies which bind to an epitope contained in the sequence SEQ ID No.3.

13. The assessment method according to claim 12, **characterized in that** the applied antibodies are polyclonal.

14. The assessment method according to claim 12, **characterized in that** the applied antibodies are monoclonal.

15. The assessment method according to any of claims 12 to 14, **characterized in that** the antibody/epitope bond is detected by an immunological technique selected from the ELISA technique, immuno-enzymatic techniques, immuno-fluorescence techniques, radio-immunological techniques, chemo-immunological techniques, and proteomic analysis techniques.

16. The assessment method according to any of claims 12 to 14, **characterized in that** the biological sample is selected from blood, serum, plasma, urine, saliva, sweat, lymph, tears, synovial liquid, tissues, cells, or supernatants of tissue or cell cultures from the individual.

17. A method for diagnosing pathology of cartilaginous tissues in an individual applying an assessment method according to any of claims 1 to 16.

18. The method according to claim 17, **characterized in that** the level of detected specific labels is quantified and compared with a reference level characteristic of the absence of pathology.

19. A method for tracking the development of a pathology of cartilaginous tissues in an individual affected by a pathology of cartilaginous tissues, applying an assessment method according to any of claims 1 to 16.

20. The method according to claim 19, **characterized in that** the level of detected specific labels is quantified and compared with a reference level for example corresponding to the level obtained for a predetermined stage of the pathology.

21. A method for prognosing in a pre-symptomatic way, a pathology of cartilaginous tissues in an individual applying an assessment method according to any of claims 1 to 16.

22. The method according to claim 21, **characterized in that** the level of detected specific labels is quantified and compared with a reference level established in individuals of the same age and of the same sex, without any pathology or at a predetermined stage of the pathology.

23. A method for predetermining the development of pathology of cartilaginous tissues in an individual affected by such a disease, applying an assessment method according to any claims 1 to 16.

24. The method according to claim 23, **characterized in that** the level of detected specific labels is quantified and compared with a reference level corresponding to a level representing the absence of the pathology or representing a predetermined stage of the pathology.

25. The method according to any of claims 17 to 24, **characterized in that** at least two specific labels of the degradation of collagen of type II is detected in the biological sample from the individual.

26. The method according to claim 23, **characterized in that** both the level of Helix-II and the level of CTX-II of sequence SEQ ID No.4 are detected in the biological sample from the individual.

27. The method according to any of claims 17 to 26, **characterized in that** the pathology of the cartilaginous tissues is inflammatory rheumatism, metabolic arthropathy, degenerative rheumatism, rheumatoid arthritis, spondylarthropathy or arthrosis.

28. The method according to claim 27, **characterized in that** the pathology of cartilaginous tissues is rheumatoid arthritis.

29. The method according to claim 27, **characterized in that** the pathology of the cartilaginous tissues is arthrosis.

30. The method according to claim 27, **characterized in that** the pathology of the cartilaginous tissues is spondylarthritis.

31. An antibody binding to an epitope for which at least four, preferably at least five, amino acids belong to the degradation fragment corresponding to the Helix-II peptide of sequence SEQ ID No.3, or to the Helix-II peptide for which one or several amino acids have been modified post-translationally and preferentially to an epitope contained in the sequence SEQ ID No.3.

32. The antibody according to claim 31, binding to an epitope for which the C-terminal portion consists of PGTS or HypGTS, possibly modified post-translationally, the C-terminal serine being preferentially not modified.

33. An antibody binding to an epitope contained in sequences SEQ ID No.3, SEQ ID No.5, SEQ ID No.6, SEQ ID No.7, or SEQ ID No.9.

34. The antibody according to any of claims 31 to 33, binding to an epitope, the sequence of which corresponds to SEQ ID No.3.

35. The antibody according to any of claims 31 to 34, **characterized in that** it is polyclonal.

36. The antibody according to any of claims 31 to 34, **characterized in that** it is monoclonal.

37. A kit for assaying a degradation fragment of collagen of type II including an antibody according to any of claims 31 to 36.

## Patentansprüche

1. Verfahren zur Einschätzung des Abbaus der Knorpelgewebe eines Individuums aus der *In-vitro*-Detektion in einer biologischen Probe, die von dem Individuum kommt, des Gehalts von wenigstens einem Marker, der spezifisch ist für den Abbau der Knorpelgewebe, **dadurch gekennzeichnet, daß** die spezifischen detektierten Marker Fragmente eines Abbaus von Typ-II-Kollagen sind, die aus dem Abbau der Aminosäuresequenz SEQ ID N° 2 der Kette α₁ des Typ-II-Kollagens stammen, welche in dem Tripelhelixbereich angeordnet ist, wobei diese Abbaufragmente wenigstens vier aufeinanderfolgende Aminosäuren umfassen, die in dem Helix-II-Peptid mit SEQ ID N° 3 vorliegen, das gegebenenfalls nach der Übersetzung modifiziert ist und in welchem das 4-Hydroxyprolin gegebenenfalls ersetzt ist durch ein Prolin oder ein 3-Hydroxyprolin.

2. Verfahren zur Einschätzung nach Anspruch 1, **dadurch gekennzeichnet, daß** die spezifischen detektierten Marker Abbaufragmente des Typ-II-Kollagens sind, die aus dem Abbau der Aminosäuresequenz SEQ ID N° 2 der α₁-Kette des Typ-II-Kollagens stammen, welche in dem Tripelhelixbereich angeordnet ist, wobei diese Abbaufragmente wenigstens vier aufeinanderfolgende Aminosäuren umfassen, die in dem Helix-II-Peptid mit SEQ ID N° 3 vorliegen, das gegebenenfalls nach der Übersetzung modifiziert ist.

3. Verfahren zur Einschätzung nach Anspruch 1 oder 2, **dadurch gekennzeichnet, daß** man den Gehalt von Abbaufragmenten der SEQ ID N° 2 des Typ-II-Kollagens detektiert, deren C-terminaler Teil besteht aus vier C-terminalen Aminosäuren des Helix-II-Peptids, nämlich Hyp-GTS, die gegebenenfalls nach der Übersetzung modifiziert sind, wobei das C-terminale Serin vorzugsweise nicht nach der Übersetzung modifiziert ist.

4. Verfahren zur Einschätzung nach einem der Ansprüche 1 bis 3, **dadurch gekennzeichnet, daß** man den Gehalt von Abbaufragmenten der SEQ ID N° 2 des Typ-II-Kollagens detektiert, welche wenigstens fünf aufeinanderfolgende Aminosäuren des Helix-II-Peptids mit SEQ ID N° 3 umfassen, von denen gegebenenfalls eine oder mehrere Aminosäuren nach der Übersetzung modifiziert worden ist (sind) und in welchem das 4-Hydroxyprolin gegebenenfalls ersetzt ist durch ein 3-Hydroxyprolin oder ein Prolin.

5. Verfahren zur Einschätzung nach einem der Ansprüche 1 bis 4, **dadurch gekennzeichnet, daß** man den Gehalt von wenigstens einem Abbaufragment des Typ-II-Kollagens detektiert, dessen Sequenz die SEQ ID N° 3 umfaßt.

6. Verfahren zur Einschätzung nach einem der Ansprüche 1 bis 5, **dadurch gekennzeichnet, daß** man den Gehalt von Helix-II mit der Sequenz SEQ ID N° 3 detektiert.

7. Verfahren zur Einschätzung nach einem der Ansprüche 1 bis 6, **dadurch gekennzeichnet, daß** die Detektion durchgeführt wird durch eine Hochleistungsflüssigchromatographietechnik (HPLC), durch Fluoreszenz, durch UV-Spektroskopie, durch elektrochemische Detektion oder durch proteomische Analysetechniken.

8. Verfahren zur Einschätzung nach einem der Ansprüche 1 bis 6, **dadurch gekennzeichnet, daß** die Detektion darin besteht, die biologische Probe mit Antikörpern, Antikörperfragmenten oder Analyten zu kontaktieren, die gegen den zu detektierenden Marker gerichtet sind, und dann deren Bindung mit dem Marker durch eine immunologische Technik zu detektieren, die vorzugsweise gewählt ist unter der ELISA-Technik, den immunoenzymatischen Techniken, den Immunofluoreszenztechniken, den Radioimmunologietechniken, den Chemoimmunologietechniken oder den Proteomanalysetechniken.

9. Verfahren zur Einschätzung nach Anspruch 8, **dadurch gekennzeichnet, daß** die biologische Probe kontaktiert wird mit polyklonalen Antikörpern.

10. Verfahren zur Einschätzung nach Anspruch 8, **dadurch gekennzeichnet, daß** die biologische Probe kontaktiert wird mit monoklonalen Antikörpern.

11. Verfahren zur Einschätzung nach einem der Ansprüche 1 bis 10, **dadurch gekennzeichnet, daß** die biologische Probe gewählt wird unter Blut, Serum, Plasma, Urin, Speichel, Schweiß, Lymphflüssigkeit, Tränen, Synovialflüssigkeit, Geweben, Zellen oder Überständen von Gewebe- oder Zellkulturen des Individuums.

12. Verfahren zur Einschätzung des Abbaus der Knorpelgewebe eines Individuums aus der *In-vitro*-Detektion in einer biologischen Probe des Individuums der Gegenwart von Abbaufragmenten des Typ-II-Kollagens, **dadurch gekennzeichnet, daß** man die biologische Probe mit Antikörpern kontaktiert, die sich an ein Epitop binden, das in der Sequenz SEQ ID N° 3 enthalten ist.

13. Verfahren zur Einschätzung nach Anspruch 12, **dadurch gekennzeichnet, daß** die eingesetzten Antikörper polyklonal sind.

14. Verfahren zur Einschätzung nach Anspruch 12, **dadurch gekennzeichnet, daß** die eingesetzten Antikörper monoklonal sind.

15. Verfahren zur Einschätzung nach einem der Ansprüche 12 bis 14, **dadurch gekennzeichnet, daß** die Bindung Antikörper/Epitop detektiert wird durch eine immunologische Technik, die gewählt ist unter der ELISA-Technik, den immunoenzymatischen Techniken, den Immunofluoreszenztechniken, den Radioimmunologietechniken, den Chemo-Immunologietechniken und den Proteomanalysetechniken.

16. Verfahren zur Einschätzung nach einem der Ansprüche 12 bis 14, **dadurch gekennzeichnet, daß** die biologische Probe gewählt wird unter Blut, Serum, Plasma, Urin, Speichel, Schweiß, Lymphflüssigkeit, Tränen, Synovialflüssigkeit, Geweben, Zellen oder Überständen von Gewebe- oder Zellkulturen des Individuums.

17. Verfahren zum Diagnostizieren einer Pathologie von Knorpelgeweben bei einem Individuum unter Einsatz eines Verfahrens zur Einschätzung nach einem der Ansprüche 1 bis 16.

18. Verfahren nach Anspruch 17, **dadurch gekennzeichnet, daß** der Gehalt detektierter spezifischer Marker quantifiziert und verglichen wird mit einem Referenzgehalt, der charakteristisch für die Pathologieabwesenheit ist.

19. Verfahren zum Verfolgen der Entwicklung einer Pathologie der Knorpelgewebe eines Individuums, das von einer Knorpelgewebepathologie betroffen ist, unter Einsatz eines Verfahrens zur Einschätzung nach einem der Ansprüche 1 bis 16.

20. Verfahren nach Anspruch 19, **dadurch gekennzeichnet, daß** der Gehalt detektierter spezifischer Marker quantifiziert und verglichen wird mit einem Referenzgehalt, der zum Beispiel dem Gehalt entspricht, der erhalten wird für ein vorbestimmtes Stadium der Pathologie.

21. Verfahren zum Prognostizieren in pre-symptomatischer Weise von einer Pathologie der Knorpelgewebe bei einem Individuum unter Einsatz eines Bewertungsverfahrens nach einem der Ansprüche 1 bis 16.

22. Verfahren nach Anspruch 21, **dadurch gekennzeichnet, daß** der Gehalt spezifischer detektierter Marker quantifiziert und verglichen wird mit einem Referenzgehalt, der etabliert ist bei Individuen selben Alters und selben Geschlechts ohne Pathologie oder bei einem vorbestimmten Stadium der Pathologie.

23. Verfahren, um die Entwicklung einer Pathologie der Knorpelgewebe bei einem Individuum vorzubestimmen, das eine solche Krankheit betroffen ist, unter Einsatz eines Verfahrens nach einem der Ansprüche 1 bis 16.

24. Verfahren nach Anspruch 23, **dadurch gekennzeichnet, daß** der Gehalt der spezifischen detektieren Marker quantifiziert und verglichen wird mit einem Referenzgehalt, der einem Gehalt entspricht, der die Abwesenheit der Pathologie darstellt oder ein vorbestimmtes Stadium der Pathologie darstellt.

25. Verfahren nach einem der Ansprüche 17 bis 24, **dadurch gekennzeichnet, daß** man in der biologischen Probe des Individuums wenigstens zwei spezifische Marker des Abbaus von Typ-II-Kollagen detektiert.

26. Verfahren nach Anspruch 23, **dadurch gekennzeichnet, daß** man in der biologischen Probe des Individuums gleichzeitig den Gehalt von Helix-II und den Gehalt von CTX-II mit Sequenz SEQ ID N° 4 detektiert.

27. Verfahren nach einem der Ansprüche 17 bis 26, **dadurch gekennzeichnet, daß** die Pathologie der Knorpelgewebe ein entzündliches Rheuma, eine metabolische Arthropatie, ein degeneratives Rheuma, eine rheumatoide Polyarthritis, eine Spondyarthropathie oder Arthrose ist.

28. Verfahren nach Anspruch 27, **dadurch gekennzeichnet, daß** die Pathologie der Knorpelgewebe die rheumatoide Polyarthritis ist.

29. Verfahren nach Anspruch 27, **dadurch gekennzeichnet, daß** die Pathologie der Knorpelgewebe Arthrose ist.

30. Verfahren nach Anspruch 27, **dadurch gekennzeichnet, daß** die Pathologie der Knorpelgewebe Spondylarthritis ist.

31. Antikörper, der sich an ein Epitop bindet, von dem wenigstens vier, vorzugsweise wenigstens fünf Aminosäuren zu dem Abbaufragment gehören, das dem Helix-II-Peptid mit Sequenz SEQ ID N° 3 oder zum Helix-II-Peptid, von dem eine oder mehrere Aminosäuren nach der Übersetzung modifiziert worden sind, und vorzugsweise einem Epitop, das in der Sequenz SEQ ID N° 3 enthalten ist, entsprechen.

32. Antikörper nach Anspruch 31, der sich an ein Epitop bindet, dessen C-terminaler Teil besteht aus PGTS oder Hyp-GTS, gegebenenfalls nach der Übersetzung modifiziert, wobei das C-terminale Serin vorzugsweise nicht modifiziert ist.

33. Antikörper, der sich an ein Epitop bindet, das in den Sequenzen SEQ ID N° 3, SEQ ID N° 5, SEQ ID N° 6, SEQ ID N° 7 oder SEQ ID N° 9 enthalten ist.

34. Antikörper nach einem der Ansprüche 31 bis 33, der sich an ein Epitop bindet, dessen Sequenz SEQ ID N° 3 entspricht.

35. Antikörper nach einem der Ansprüche 31 bis 34, **dadurch gekennzeichnet, daß** er polyklonal ist.

36. Antikörper nach einem der Ansprüche 31 bis 34, **dadurch gekennzeichnet, daß** er monoklonal ist.

37. Dosierkit eines Abbaufragments des Typ-II-Kollagens, umfassend einen Antikörper nach einem der Ansprüche 31 bis 36.
